# EUROPEAN PATENT APPLICATION

(11) **EP 1 579 878 A1**
(43) Date of publication of application: **28.09.2005**
(21) Application number: 05251851.1
(22) Date of filing: 24.03.2005
(51) Int. Cl.: A61L 31/18, A61B 10/00

(54) **Apparatus and method for marking tissue**

(30) Priority: 26.03.2004 US 810354
(71) Applicant: ETHICON ENDO-SURGERY, INC., Cincinnati, Ohio 45242 (US)
(72) Inventor: Beckman, Andrew T., Cincinnati, Ohio 45245 (US); Onvumere, Fidelis, 19107 Mansfield, Texas 76063 (US); Ludzack, Michael Robert, Maineville, Ohio 45039 (US); Samples, Charles Robert, Akron, Ohio 44319 (US); Barbur, Ana, Hudson, Ohio 44236 (US)
(74) Representative: Mercer, Christopher Paul

(57) **Abstract**

The present invention includes methods and materials for implantable devices (markers) which are disclosed for permanently marking the location of a biopsy or surgery for the purpose of identification. The devices are remotely delivered, preferably percutaneously. Visualization of the markers is readily accomplished using various state of the art imaging systems. Preferred visualization is through MRI, X-ray and ultrasound. The markers function to provide evidence of the location of the lesion after the procedure is complete for reference during future examinations or procedures.

## Description

### FIELD OF THE INVENTION

The present invention relates to markers to be employed at biopsy sites to permanently mark the site, and to methods and apparatus for applying the permanent marker. More particularly, the present invention relates to a marker that is optimally adapted for marking biopsy sites in human breast tissue with permanently placed markers that are detectable by MRI, ultrasound and X-ray. This invention relates to methods and devices for marking and defining particular locations in body tissue, particularly human tissue, and more particularly relates to methods and devices for permanently defining the location and margins of lesions detected in biopsy cavity walls.

### BACKGROUND OF THE INVENTION

In the U.S. alone approximately one million women will have breast biopsies because of irregular mammograms and palpable abnormalities. Biopsies can include surgical excisional biopsies and stereotactic and ultrasound guided needle breast biopsies. In the case of image directed biopsy, the radiologist or other physician takes a small sample of the irregular tissue for laboratory analysis. If the biopsy proves to be malignant, additional surgery (typically a lumpectomy or a mastectomy) is required. In the case of needle biopsies, the patient then returns to the radiologist a day or two later where the biopsy site (the site of the lesion) is relocated by method called needle localization, a preoperative localization in preparation for the surgery.

A biopsy may be an open or percutaneous technique. Open biopsy removes the entire mass (excisional biopsy) or a part of the mass (incisional biopsy). Percutaneous biopsy on the other hand is usually done with a needle-like instrument and may be either a fine needle aspiration (FNA) or a core biopsy. In FNA biopsy, very small needles are used to obtain individual cells or clusters of cells for cytologic examination. The cells may be prepared such as in a Papanicolaou (Pap) smear. In core biopsy, as the term suggests, a core or fragment of tissue is obtained for histologic examination, which may be done via a frozen section or paraffin section. The chief difference between FNA and core biopsy is the size of the tissue sample taken. A real time or near real time imaging system having stereoscopic capabilities, such as the stereotactic guidance system described in U.S. Pat. No. 5,240,011, is employed to guide the extraction instrument to the lesion. Advantageous methods and devices for performing core biopsies are described in U.S. Pat. No. 5,526,822.

Depending upon the procedure being performed, it is sometimes desirable to completely remove suspicious lesions for evaluation, while in other instances it may be desirable to remove only a sample from the lesion. In the former case, a major problem is the ability to define the margins of the lesions at all times during the extraction process. Visibility of the lesion by the imaging system may be hampered because of the distortion created by the extraction process itself as well as associated bleeding in the surrounding tissues. Although the lesion is removed and all fluids are continuously aspirated from the extraction site, it is likely that the process will "cloud" the lesion, thus impairing exact recognition of its margins. This makes it difficult to ensure that the entire lesion will be removed.

Often, the lesion is merely a calcification derived from dead abnormal tissue, which may be cancerous or pre-cancerous, and it is desirable to remove only a sample of the lesion, rather than the entire lesion, to evaluate it. This is because such a lesion actually serves to mark or define the location of adjacent abnormal tissue, so the physician does not wish to remove the entire lesion and thereby lose a critical means for later re-locating the affected tissue. One of the benefits to the patient from core biopsy is that the mass of the tissue taken is relatively small. However, oftentimes, either inadvertently or because the lesion is too small, the entire lesion is removed for evaluation, even though it is desired to remove only a portion. Then, if subsequent analysis indicates the tissue to be malignant (malignant tissue requires removal, days or weeks later, of tissue around the immediate site of the original biopsy), it is difficult for the physician to determine the precise location of the lesion, in order to perform necessary additional procedures on adjacent potentially cancerous tissue. Additionally, even if the lesion is found to be benign, there will be no evidence of its location during future examinations to mark the location of the previously removed calcification so that the affected tissue may be carefully monitored for future re-occurrences.

Thus, it would be of considerable benefit to be able to permanently mark the location or margins of such a lesion prior to or immediately after removing or sampling it. Marking prior to removal would help to ensure that the entire lesion is excised, if desired. Alternatively, if the lesion were inadvertently removed in its entirety, marking the biopsy site immediately after the procedure would enable reestablishment of its location for future identification.

A number of procedures and devices for marking and locating particular tissue locations are known in the prior art. For example, location wire guides, such as that described in U.S. Pat. No. 5,221,269 to Miller *et al*., are well known for locating lesions, particularly in the breast. The device described by Miller comprises a tubular introducer needle and an attached wire guide, which has at its distal end a helical coil configuration for locking into position about the targeted lesion. The needle is introduced into the breast and guided to the lesion site by an imaging system of a known type, for example, x-ray, ultrasound, or magnetic resonance imaging (MRI), at which time the helical coil at the distal end is deployed about the lesion. Then, the needle may be removed from the wire guide, which remains in a locked position distally about the lesion for guiding a surgeon down the wire to the lesion site during subsequent surgery. While such a location system is effective, it is obviously intended and designed to be only temporary, and is removed once the surgery or other procedure has been completed.

Other devices are known for marking external regions of a patient's skin. For example, U.S. Pat. No. 5,192,270 discloses a syringe that dispenses a colorant to give a visual indication on the surface of the skin of the point, at which an injection has or will be given. Similarly, U.S. Pat. No. 5,147,307 discloses a device that has patterning elements for impressing a temporary mark in a patient's skin, for guiding the location of an injection or the like. It is also known to tape or otherwise adhere a small metallic marker, e.g., a 3 millimeter diameter lead sphere on the skin of a human breast in order to delineate the location of skin calcifications (see Homer *et al.,* The Geographic Cluster of Microcalcifications of the Breast, Surgery, Gynecology, & Obstetrics, December 1985). Obviously, however, none of these approaches are useful for marking and delineating internal tissue abnormalities, such as lesions or tumors.

Still another approach for marking potential lesions and tumors of the breast is described in U.S. Pat. No. 4,080,959. In the described procedure, the skin of the portion of the body to be evaluated, such as the breasts, is coated with a heat sensitive color-responsive chemical, after which that portion of the body is heated with penetrating radiation such as diathermy. Then, the coated body portion is scanned for color changes that would indicate hot spots beneath the skin surface. These so-called hot spots may represent a tumor or lesion, which does not dissipate heat as rapidly because of its relatively poor blood circulation (about 1/20 of the blood flow through normal body tissue). This method, of course, functions as a temporary diagnostic tool, rather than a permanent means for delineating the location of a tumor or lesion.

A method of identifying and treating abnormal neoplastic tissue or pathogens within the body is described in U.S. Pat. No. 4,649,151. In this method, a tumor-selective photosensitizing drug is introduced into a patient's body, where it is cleared from normal tissue faster than it is cleared from abnormal tissue. After the drug has cleared normal tissue but before it has cleared abnormal neoplastic tissue, the abnormal neoplastic tissue may be located by the luminescence of the drug within the abnormal tissue. The fluorescence may be observed with low intensity light, some of which is within the drug's absorbance spectrum, or higher intensity light, a portion of which is not in the drug's absorbance spectrum. Once detected, the tissue may be destroyed by further application of higher intensity light having a frequency within the absorbance spectrum of the drug. Of course, this method also is only a temporary means for marking the abnormal tissue, since eventually the drug will clear from even the abnormal tissue. Additionally, once the abnormal tissue has been destroyed during treatment, the marker is destroyed as well.

It is also known to employ biocompatible dyes or stains to mark breast lesions. First, a syringe containing the colorant is guided to a detected lesion, using an imaging system. Later, during the extraction procedure, the surgeon harvests a tissue sample from the stained tissue. However, while such staining techniques can be effective, it is difficult to precisely localize the stain. Also, the stains are difficult to detect fluoroscopically and may not always be permanent.

Additionally, it is known to implant markers directly into a patient's body using invasive surgical techniques. For example, during a coronary artery bypass graft (CABG), which of course constitutes open-heart surgery, it is common practice to surgically apply one or more radiopaque rings to the aorta at the site of the graft. This enables a practitioner to later return to the site of the graft by identifying the rings, for evaluative purposes. It is also common practice to mark a surgical site with staples, vascular clips, and the like, for the purpose of future evaluation of the site.

A technique has been described for the study of pharyngeal swallowing in dogs, which involves permanently implanting steel marker beads in the submucosa of the pharynx (S. S. Kramer *et al.,* A Permanent Radiopaque Maker Technique for the Study of Phalynged Swallowing in Dogs, Dysphagia, Vol. 1, pp. 163-167, 1987). The article posits that the radiographic study of these marker beads during swallowing, on many occasions over a substantial period of time, provides a better understanding of the pharyngeal phase of degluitition in humans. In the described technique, the beads were deposited using a metal needle cannula having an internal diameter slightly smaller than the beads to be implanted. When suction was applied to the cannula, the bead sat firmly on the tip. Once the ball-tipped cannula was inserted through tissue, the suction was broken, thereby releasing the bead, and the cannula withdrawn.

The concept of injecting hydrogels to fill spaces or tracks is described in U.S. Pat. No. 5,645,583 to Villain *et al.* That patent describes a polyethylene oxide gel implant that may be injected into a human body for tissue replacement and augmentation. U.S. Pat. No. 5,090,955 describes the use of gels in ophthalmology for corneal tissue augmentation procedures such as Gel Injection Adjustable Keratoplasty (GIAK). Neither patent mentions the augmentation of such tissue by hydration and swelling-induced shape changes in the tissue. Instead, for example, the Simon patent describes "smoothing and massaging" of the cornea to remove excess hydrogel material.

Non-degradable hydrogels made from poly(vinyl pyrrolidone) and methacrylate have been fashioned into fallopian tubal occluding devices that swell and occlude the lumen of the tube. See, Brundin, "Hydrogel tubal blocking device: P-Block", in Female Transcervical Sterilization, (Zatuchini *et al.,* Eds.) Harper Row, Philadelphia (1982), pp. 240-244. Because such hydrogels undergo a relatively small amount of swelling and are not absorbable, so that the sterilization is not reversible, the devices described in the foregoing reference have found limited utility.

Accordingly, what is needed is a method and device for implanting potentially permanent markers at the situs of a lesion or other abnormal tissue, for the purpose of defining the margins of a lesion before it is removed and/or to establish its location after it has been removed. The markers should be easy to deploy and easily detected using state of the art imaging techniques.

### SUMMARY OF THE INVENTION

The invention features devices and methods for a marker that has the ability to be visible under x-ray, MRI and ultrasound. This marker made of a permanent material that is retains an imaging ability under ultrasound while providing a low shadowing profile so that features are not masked behind the marker.

An implantable marking device is provided which is designed to percutaneously deliver permanent markers to desired tissue locations within a patient's body. This provides several advantages to the physician in diagnosis and management of tissue abnormalities, such as a means of localization of a tissue abnormality for follow-up surgical treatment, and a means of tissue abnormality site identification for purposes of ongoing diagnostic follow-up. In one preferred construction, a radiographic clip is configured in the form of a surgical staple. A disposable tissue marker applier, which comprises a flexible tube, pull wire, and squeeze handle, is employed to advance and deploy the clip to a desired tissue location. Either a flexible or a rigid introducer is also provided for providing access to the site to be marked.

This invention solves many of the problems in the art by providing an implantable marking device which is designed to percutaneously (through the skin) deliver permanent markers to desired tissue locations within a patient's body, even if the desired locations are laterally disposed relative to the distal end of the delivery device, as is the case for conduit or cavity walls. The device allows the physician to accurately position and deploy a marker at the site of a biopsy. This provides several advantages to the physician in diagnosis and management of tissue abnormalities, such as a means of localization of a tissue abnormality for follow-up surgical treatment, and a means of tissue abnormality site identification for purposes of ongoing diagnostic follow-up. It may also prevent inadvertent repeat biopsy of a lesion if the patient were to move or if adequate records did not follow the patient. The inventive system also represents a less traumatic means for tissue marking and a reduced procedural duration relative to the standard open surgical method.

Current ultrasound visible x-ray markers utilize absorbable materials that become non-detectable after a period of time. These typically include an embedded metallic (e.g., titanium) clip to gain x-ray visibility. The majority of markers utilize collagen as the absorbable body of the marker. Some provide a synthetic absorbable material that has minimal expansion. It is assumed that this may be the reason a plurality of markers are deployed in a single site to gain enough mass for the ultrasound visibility. The present invention provides a permanent marker material that is echogenic and radiopaque when place within the biopsy cavity.

Examples of synthetic non-biodegradable polymers, include, but are not limited to, various polyacrylates, ethylene-vinyl acetates (and other acyl-substituted cellulose acetates), polyurethanes, polystyrenes, polyvinyl oxides, polyvinyl fluorides, poly(vinyl imidazoles), chlorosulphonated polyolefins, polyethylene oxides, polyvinyl alcohols (PVA), polytetrafluoroethylenes and nylons.

A particularly preferred material for use in the device is polyvinyl alcohol (PVA) and alkylated or acylated derivatives thereof. In one embodiment, polymers can be provided in the form of expandable foam using conventional foam generation techniques available in the art. Generally, the PVA must be crosslinked by gamma radiation. This process provides structural integrity, expansion rate and softness.

In one embodiment, the present invention utilizes a permanent material that changes size and/or shape upon delivery to the biopsy cavity. The change may expand to fill the biopsy cavity. The marker also includes a permanent metallic clip that is distinctly different or obvious when visualize under x-ray.

In one embodiment, the marker is configured as an injectable polymer that expands upon entry into the biopsy cavity. In an alternate embodiment, this expansion is due to the reaction of two or more material polymers or due to the reaction to body fluids.

In another embodiment, the marker is configured as a solid polymer that is initially hard and over time expands becoming a gel like material.

In another embodiment, the marker is configured as a compressed foam that is initially hard and over time expands becoming a gel-like material.

In another embodiment, the marker is a permanent injectable polymer that is in situ expandable. Such in situ expandable marker may be a synthetic polymer latex such as isoprene, nitrile, butyl, TPE with a melting point of about 38°C to about 45°C, and hydrolyzed polyvinyl acetate (chewing gum base).

In another embodiment, the marker may be composed of at least a two part injectable substance that is mixed during the delivery to the biopsy cavity including a radiopaque detectable artifact embedded within.

In another embodiment, the marker has a radiopaque artifact is of a shape that is distinctly obvious when visualized. In another embodiment, the marker is composed of expandable material that expands to a predetermined shape that is distinctly obvious when visualized. Preferably, the expandable material expands to fill the cavity marking the biopsy cavity boundaries.

In another embodiment, the marker is permanent injectable polymer microspheres with a synthetic latex binder. In another embodiment, the marker is a permanent injectable polymer gel, sponge or foam (expandable). This can be an acrylic hydrogel (as used in reconstructive plastic surgery) or a temperature or pH sensitive gel.

In another embodiment, the marker is a permanent implantable elastomer. The elastomer material may be hard in nature prior to injection and expand post injection filling the biopsy cavity. Generally, the elastomer post deliver absorbs cavity fluids expanding and softening to mimic the surrounding tissue. The expansion of marker take a predetermined shape that is distinctly obvious when visualized using at least two detection methods.

In another embodiment, the marker may be a gelatin encapsulated permanent compressed foam or reactable co-polymers. In another embodiment, the marker may be an injectable dry polymer, e.g., Kraton 1107 plus Vistanex PIB pVAC (chewing gum).

In the present invention, preferably the polymer material is a two-part hydrogel material that is blended at the time of injection to a biopsy site.

Preferably the device of the invention additionally comprises an active agent for delivery at a biopsy site.

Preferably the active agent comprises at least one agent selected from the group consisting of a chemotherapeutic agent, a radiation agent and a gene therapy agent.

Preferably the device of the invention additionally comprises at least one agent selected from the group consisting of a pain killing substance, a hemostatic substance, an antibiotic, and a radioactive material.

Preferably the polymer is of a different hardness in the post-delivery state as in the pre-delivery state.

Preferably the polymer has a hardness of about 0.5 times to about 1.5 times as hard as breast tissue in the post-delivery state.

Preferably the polymer swells about 50 to 1500 percent from the pre-delivery state to the post-delivery state when placed in contact with an aqueous liquid.

Preferably the polymer has a first shape in the pre-delivery state and a second, predetermined shape in the post-delivery state.

Preferably the polymer has one consistency in the pre-delivery state and a different consistency in the post-delivery state.

Preferably the X-ray detectable object comprises a wire.

Preferably the object has a distinguishing shape.

Preferably the object is fixedly attached to the at least one body.

Preferably the object is radioactive.

Preferably the at least one body is radioactive.

Preferably the biocompatible material further comprises a bio-resorbable polymeric material.

Preferably the bio-resorbable polymeric material is selected from the group consisting of poly(esters), poly(hydroxy acids), poly(lactones), poly(amides), poly(ester-amides), poly(amino acids), poly(anhydrides), poly(ortho-esters), poly(carbonates), poly(phosphazines), poly(thioesters), poly(urethanes), poly(ester urethanes), polysaccharides, polylactic acids, polyglycolic acids, polycaproic acids, polybutyric acids, polyvaleric acids, and copolymers, polymer alloys, polymer mixtures, and combinations thereof.

Preferably the bio-resorbable polymeric material has a bulk density of between about 0.8 g/ml and about 1.5 g/ml.

Preferably the device of the invention further comprises a binding agent.

Preferably the binding agent is selected from the group consisting of gelatin, polyethylene glycol, polyvinyl alcohol, glycerin, acrylic hydrogels, organic hydrogels, and combinations thereof.

Preferably the binding agent comprises gelatin selected from the group consisting of bovine collagen, porcine collagen, ovine collagen, equine collagen, synthetic collagen, agar, synthetic gelatin, and combinations thereof.

The present invention also provides an implantable biopsy cavity marking device comprising at least one body comprising a resilient biocompatible polymeric material encapsulated within a biodegradable shell wherein the shell degrades upon contact with a liquid and wherein the marking device is radiopaque and echogenic.

Preferably the polymeric material comprises a non-bioabsorbable material.

Preferably the polymeric material within the shell is compressed foam or is a material selected from the group consisting of materials reactive with body fluids, liquids, binding agents, active agents or combinations thereof.

Preferably the marking device further comprises an X-ray detectable object of specific predetermined non-biological configuration embedded in the body of the marking device.

Preferably the marking device further comprises a radiopaque additive selected from the group consisting of barium-containing compounds, bismuth-containing compounds, powdered tantalum, powdered tungsten, barium carbonate, bismuth oxide, and barium sulfate.

Preferably the polymeric material is one or more polymers selected form the group consisting of polyacrylates, ethylene-vinyl acetate polymers, non-erodible polyurethanes, polystyrenes, polyvinyl chloride, polyvinyl fluoride, poly(vinyl imidazole), chlorosulphonated polyolifins, polyethylene oxide, polyvinyl alcohol, teflon, calcium carbonate, carrageenan and nylon, and derivatives thereof.

Preferably the polymeric material is selected form the group consisting of a polyvinyl alcohol gel, foam or sponge, hydrogel, and esters and acylation derivatives thereof.

Preferably the polymer is a hydrogel selected from the group consisting of a crosslinked polyethylene oxide, polypropylene oxide, polyvinyl alcohol, polyvinyl acetate, polyvinyl pyrrolidone, polyhydroxyalkyl acrylate, polystyrene sulfonate and copolymers or combinations thereof.

Preferably the shell is a layer of bioabsorbable material.

Preferably the bio-resorbable polymeric material is selected from the group consisting of collagen, cross-linked collagen, regenerated cellulose, synthetic polymers, synthetic proteins, and combinations thereof.

Preferably the bio-resorbable polymeric material is selected from the group consisting of poly(esters), poly(hydroxy acids), poly(lactones), poly(amides), poly(ester-amides), poly(amino acids), poly(anhydrides), poly(ortho-esters), poly(carbonates), poly(phosphazines), poly(thioesters), poly(urethanes), poly(ester urethanes), polysaccharides, polylactic acids, polyglycolic acids, polycaproic acids, polybutyric acids, polyvaleric acids, and copolymers, polymer alloys, polymer mixtures, and combinations thereof.

Preferably the polymer material is a two-part hydrogel material.

Preferably the device additionally comprises an active agent for delivery at a biopsy site.

Preferably the active agent comprises at least one agent selected from the group consisting of a chemotherapeutic agent, a radiation agent and a gene therapy agent.

Preferably the polymeric material is of a different hardness in the post-delivery state as in the pre-delivery state.

Preferably the polymeric material has a hardness of about 0.5 times to about 1.5 times as hard as breast tissue in the post-delivery state.

Preferably the polymeric material swells about 50 to 1500 percent from the pre-delivery state to the post-delivery state when placed in contact with a liquid.

Preferably the polymer has a first shape in the pre-delivery state and a second shape in the post-delivery state.

Preferably the polymer has one consistency in the pre-delivery state and a different consistency in the post-delivery state.

Preferably the X-ray detectable object comprises a wire.

Preferably the object has a distinguishing shape.

Preferably the object is fixedly attached to the at least one body.

Preferably the object is radioactive.

Preferably the at least one body is radioactive.

Preferably the biocompatible material further comprises a bio-resorbable polymeric material.

Preferably the bio-resorbable polymeric material is selected from the group consisting of poly(esters), poly(hydroxy acids), poly(lactones), poly(amides), poly(ester-amides), poly(amino acids), poly(anhydrides), poly(ortho-esters), poly(carbonates), poly(phosphazines), poly(thioesters), poly(urethanes), poly(ester urethanes), polysaccharides, polylactic acids, polyglycolic acids, polycaproic acids, polybutyric acids, polyvaleric acids, and copolymers, polymer alloys, polymer mixtures, and combinations thereof.

Preferably the bio-resorbable polymeric material has a bulk density of between about 0.8 g/ml and about 1.5 g/ml.

Preferably the device further comprises a binding agent.

Preferably the binding agent is selected from the group consisting of gelatin, polyethylene glycol, polyvinyl alcohol, glycerin, acrylic hydrogels, organic hydrogels, and combinations thereof.

Preferably the binding agent comprises gelatin selected from the group consisting of bovine collagen, porcine collagen, ovine collagen, equine collagen, synthetic collagen, agar, synthetic gelatin, and combinations thereof.

The present invention also provides a method of (or an apparatus for) marking a biopsy site within a subject's body, comprising (means for) depositing an implantable biopsy cavity marking device comprising at least one body comprising a resilient biocompatible material, wherein the marking device is radiopaque and echogenic.

Preferably the at least one body comprises a non-bioabsorbable material.

Preferably the marking device further comprises an X-ray detectable object of specific predetermined non-biological configuration embedded in the body of the marking device.

Preferably the marker comprises a material selected from the group consisting of platinum, iridium, nickel, tungsten, tantalum, gold, silver, rhodium, titanium, alloys thereof, and stainless steel.

Preferably the biocompatible material comprises a polymer.

Preferably the polymer is one or more polymers selected form the group consisting of polyacrylates, ethylene-vinyl acetate polymers, non-erodible polyurethanes, polystyrenes, polyvinyl chloride, polyvinyl fluoride, poly(vinyl imidazole), chlorosulphonated polyolifins, polyethylene oxide, polyvinyl alcohol, teflon, calcium carbonate, carrageenan and nylon, and derivatives thereof.

Preferably the polymer is a polyvinyl alcohol gel, foam, sponge, swellable polymer, hydrogels and acylation derivatives thereof, including esters.

Preferably the polymer is a hydrogel selected from the group consisting of a crosslinked polyethylene oxide, polypropylene oxide, polyvinyl alcohol, polyvinyl acetate, polyvinyl pyrrolidone, polyhydroxyalkyl acrylate, polystyrene sulfonate and copolymers or combinations thereof.

Preferably the material is effective to form a gel upon introduction within the body of an animal.

Preferably the material forms a gel upon introduction within the body of an animal after contact with a biocompatible liquid.

Preferably the biocompatible liquid comprises a hemostatic agent selected from the group consisting of tissue fluid, water, binding agents, active agents, liquid polymers, hemostatic agents.

Preferably the biocompatible liquid comprises a pharmaceutical agent selected from the group consisting of penicillins, cephalosporins, vancomycins, aminoglycosides, quinolones, polymyxins, erythromycins, tetracyclines, streptomycins, sulfa drugs, chloramphenicols, clindamycins, lincomycins, sulfonamides, paclitaxel, docetaxel, acetyl sulfisoxazole, alkylating agents, antimetabolites, plant alkaloids, mechlorethamine, chlorambucil, cyclophosphamide, melphalan, ifosfamide, methotrexate, 6-mercaptopurine, 5-fluorouracil, cytarabine, vinblastine, vincristine, etoposide, doxorubicin, daunomycin, bleomycin, mitomycin, carmustine, lomustine, cisplatin, interferon, asparaginase, tamoxifen, flutamide, amantadines, rimantadines, ribavirins, idoxuridines, vidarabines, trifluridines, acyclovirs, ganciclovirs, zidovudines, foscarnets, interferons, prochlorperzine edisylate, ferrous sulfate, aminocaproic acid, mecamylamine hydrochloride, procainamide hydrochloride, isoproterenol sulfate, phenmetrazine hydrochloride, bethanechol chloride, methacholine chloride, isopropamide iodide, tridihexethyl chloride, phenformin hydrochloride, methylphenidate hydrochloride, theophylline cholinate, cephalexin hydrochloride, diphenidol, meclizine hydrochloride, prochlorperazine maleate, phenoxybenzamine, thiethylperzine maleate, anisindone, diphenadione erythrityl tetranitrate, isoflurophate, acetazolamide, methazolamide, bendroflumethiazide, chloropromaide, tolazamide, chlormadinone acetate, phenaglycodol, allopurinol, aluminum aspirin, hydrocortisone, hydrocorticosterone acetate, cortisone acetate, dexamethasone and its derivatives such as betamethasone, triamcinolone, methyltestosterone, 17-S-estradiol, ethinyl estradiol, ethinyl estradiol 3-methyl ether, prednisolone, 17-hydroxyprogesterone acetate compounds, 19-nor-progesterone, norgestrel, norethindrone, norethisterone, norethiederone, progesterone, norgesterone, norethynodrel, aspirin, indomethacin, naproxen, fenoprofen, sulindac, indoprofen, nitroglycerin, isosorbide dinitrate, propranolol, timolol, atenolol, alprenolol, cimetidine, clonidine, imipramine, dihydroxyphenylalanine, theophylline, calcium gluconate, ketoprofen, ibuprofen, cephalexin, haloperidol, zomepirac, ferrous lactate, vincamine, diazepam, phenoxybenzamine, milrinone, capropril, mandol, quanbenz, hydrochlorothiazide, ranitidine, flurbiprofen, fenufen, fluprofen, tolmetin, alclofenac, mefenamic, flufenamic, difuinal, nizatidine, sucralfate, etintidine, tetratolol, minoxidil, chlordiazepoxide, diazepam, amitriptyline, imipramine, prostaglandins, coagulation factors, analogs of these compounds, derivatives of these compounds, and pharmaceutically acceptable salts of these compounds, analogs and derivatives.

Preferably the biocompatible liquid comprises a hemostatic agent selected from the group consisting of adrenochrome, algin, alginic acid, aminocaproic acid, batroxobin, carbazochrome salicylate, cephalins, cotarmine, ellagic acid, epinephrine, ethamsylate, factor VIII, factor IX, factor XIII, fibrin, fibrinogen, naphthoquinone, oxamarin, oxidized cellulose, styptic collodion, sulamrin, thrombin, thromboplastin (factor III), tolonium chloride, tranexamic acid, and vasopression.

Preferably the quantity of ultrasound-detectable material comprises a slurry of ultrasound-detectable material in a biocompatible liquid.

Preferably the slurry is formed within a delivery tube.

Preferably the slurry is formed within a syringe.

Preferably the device is (or is adapted to be) positioned by a positioning step carried out by at least one of: injecting a flowable polymer through a hollow member; pushing a nonflowable polymer through a hollow member; and guiding a solid polymer to the target site.

Preferably the flowable polymer injecting step is carried out using a biopsy needle.

The method (or apparatus) preferably further comprises the step of (or means for) changing the polymer from a pre-delivery state prior to the positioning step to a post-delivery state after the positioning step.

Preferably the changing step is carried out by at least one of the following: hydration, changing temperature, electrical stimulation, magnetic stimulation, chemical reaction with a first additional material, physical interaction with a second additional material, ionization, absorption and adsorption.

Preferably the method (or apparatus) further comprises the step of (or means for) placing a marker element at a generally central location within the polymer at the target site.

Preferably the biopsy site relocating step comprises the step of remotely visualizing the marker element.

Preferably the method further comprises: testing the tissue sample and, if the testing indicates a need to do so, medically treating the biopsy site.

Preferably the medically treating step comprises activating an agent carried by the polymer.

Preferably the activating step is carried out by at least one of: injecting a radiation-emitting element at the vicinity of the target site; externally irradiating the target site; and providing a triggering substance to the agent.

Preferably the medically treating step comprises delivering a therapeutic agent to the target site.

Preferably the delivering step is carried out using at least one of: a chemotherapy agent; a radiation-emitting element; thermal energy; ionization energy; gene therapy; vector therapy; electrical therapy; vibrational therapy; and anti-angiogenesis.

Preferably the method (or apparatus) further comprises the step of (or means for) relocating the biopsy by fmding the polymer.

Preferably the medical treating step comprises removal of tissue.

Preferably the marking device comprises at least one body comprising a resilient biocompatible polymeric material encapsulated within a biodegradable shell wherein the shell.

Preferably the shell is a layer of bioabsorbable material that degrades upon contact with a liquid.

Preferably the bio-resorbable polymeric material is selected from the group consisting of collagen, cross-linked collagen, regenerated cellulose, synthetic polymers, synthetic proteins, and combinations thereof.

Preferably the bio-resorbable polymeric material is selected from the group consisting of poly(esters), poly(hydroxy acids), poly(lactones), poly(amides), poly(ester-amides), poly(amino acids), poly(anhydrides), poly(ortho-esters), poly(carbonates), poly(phosphazines), poly(thioesters), poly(urethanes), poly(ester urethanes), polysaccharides, polylactic acids, polyglycolic acids, polycaproic acids, polybutyric acids, polyvaleric acids, and copolymers, polymer alloys, polymer mixtures, and combinations thereof.

Preferably the marking device further comprises a binding agent.

Preferably the binding agent is selected from the group consisting of gelatin, polyethylene glycol, polyvinyl alcohol, glycerin, acrylic hydrogels, organic hydrogels, and combinations thereof.

Preferably the binding agent comprises gelatin selected from the group consisting of bovine collagen, porcine collagen, ovine collagen, equine collagen, synthetic collagen, agar, synthetic gelatin, and combinations thereof.

In addition to the above features, ultrasound-detectable biopsy marker materials embodying features of the invention may also include radiopaque materials or radiopaque elements, so that the biopsy site may be detected both with ultrasound and with X-ray or other radiographic imaging techniques. Radiopaque materials and markers may include metal objects such as clips, bands, strips, coils, and other objects made from radiopaque metals and metal alloys, and may also include powders or particulate masses of radiopaque materials. Radiopaque markers may be of any suitable shape or size, and are typically formed in a recognizable shape not naturally found within a patient's body, such as a star, square, rectangular, geometric, gamma, letter, coil or loop shape. Suitable radiopaque materials include stainless steel, platinum, gold, iridium, tantalum, tungsten, silver, rhodium, nickel, bismuth, other radiopaque metals, alloys and oxides of these metals, barium salts, iodine salts, iodinated materials, and combinations of these. Radiopaque materials and markers may be permanent, or may be temporary and not detectable after a period of time subsequent to their placement at a biopsy site within a patient.

In addition, ultrasound-detectable biopsy marker materials embodying features of the invention may also include MRI-detectable materials or markers, so that the biopsy site may be detected both with ultrasound and with MRI or other imaging techniques. MRI contrast agents such as gadolinium and gadolinium compounds, for example, are suitable for use with ultrasound-detectable biopsy marker materials embodying features of the invention. Colorants, such as dyes (e.g., methylene blue and carbon black) and pigments (e.g., barium sulfate), may also be included in ultrasound-detectable biopsy marker materials embodying features of the invention.

An advantage of an injectable marker of the present invention is that such markers can be deployed using various surgical needles and various biopsy device sizes for placement at the biopsy site. This universal marker eliminates the need for multiple marker product specifically designed for each needle size. The injection marker type can be controlled injection related to the biopsy taken or physician preference.

Throughout this document, all temperatures are given in degrees Celsius, and all percentages are weight percentages unless otherwise stated. Unless defined otherwise, all technical and scientific terms used herein have the same meaning as commonly understood to one of ordinary skill in the art to which this invention belongs. Although any methods, devices and materials similar or equivalent to those described herein can be used in the practice or testing of the invention, the preferred methods, devices and materials are now described.

All publications mentioned herein are incorporated herein by reference for the purpose of describing and disclosing the compositions and methodologies which are described in the publications which might be used in connection with the presently described invention. The publications discussed herein are provided solely for their disclosure prior to the filing date of the present application. Nothing herein is to be construed as an admission that the invention is not entitled to antedate such a disclosure by virtue of prior invention. The above summary of the present invention is not intended to describe each embodiment or every implementation of the present invention.

### BRIEF DESCRIPTION OF THE DRAWINGS

This invention, as defined in the claims, can be better understood with reference to the following drawings. The drawings are not necessarily to scale, emphasis instead being placed upon clearly illustrating principles of the present invention.

The novel features of the invention are set forth with particularity in the appended claims. The invention itself, however, both as to organization and methods of operation, together with further objects and advantages thereof, may best be understood by reference to the following description, taken in conjunction with the accompanying drawings in which:

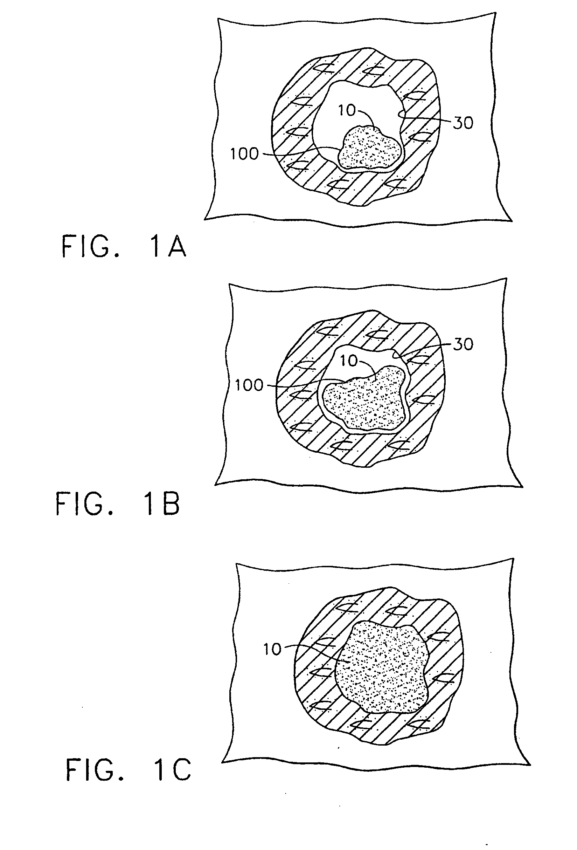

FIG. 1 illustrates a cavity marking device having the ability to swell within the biopsy cavity where (A) the marker is placed into the biopsy cavity, (B) the marker swells upon contact with a liquid, and (C) the marker takes on the shape of the biopsy cavity.

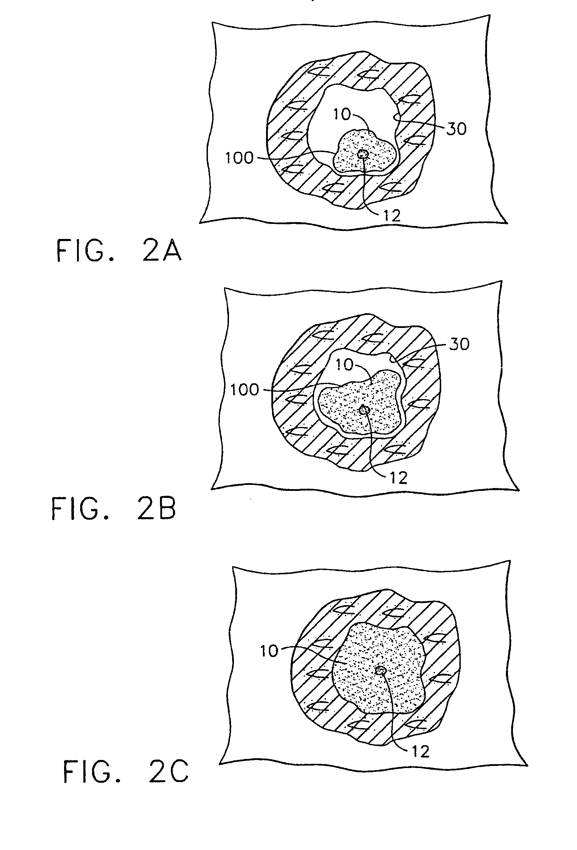

FIG. 2 illustrates a cavity marking device containing an x-ray detectable metallic marker embedded in the device wherein the device has the ability to swell within the biopsy cavity where (A) the marker is placed into the biopsy cavity, (B) the marker swells upon contact with a liquid, and (C) the marker takes on the shape of the biopsy cavity.

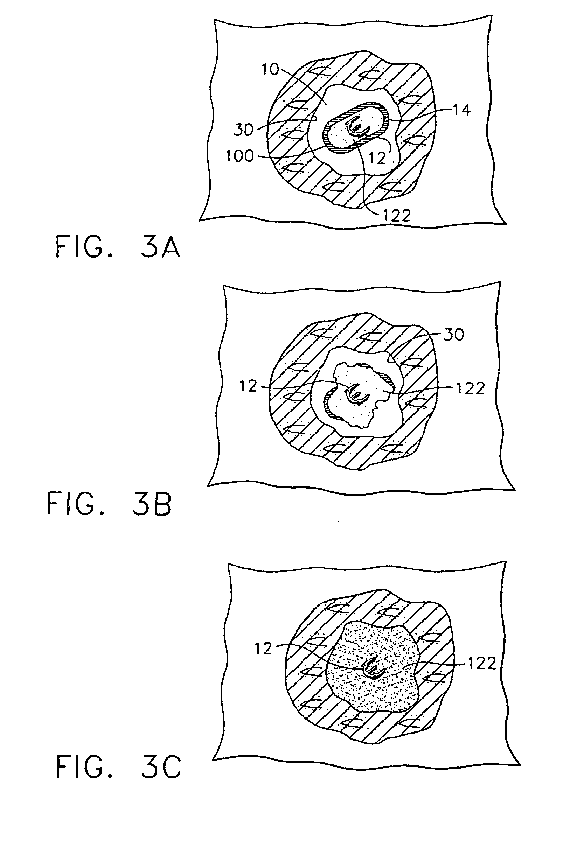

FIG. 3 illustrates a cavity marking device having the ability to swell within the biopsy cavity where the device is a polymeric material encapsulated within a biodegradable shell where (A) the marker is placed into the biopsy cavity, (B) the shell degrades upon contact with liquid within the site and the marker swells upon contact with a liquid, and (C) the marker takes on the shape of the biopsy cavity.

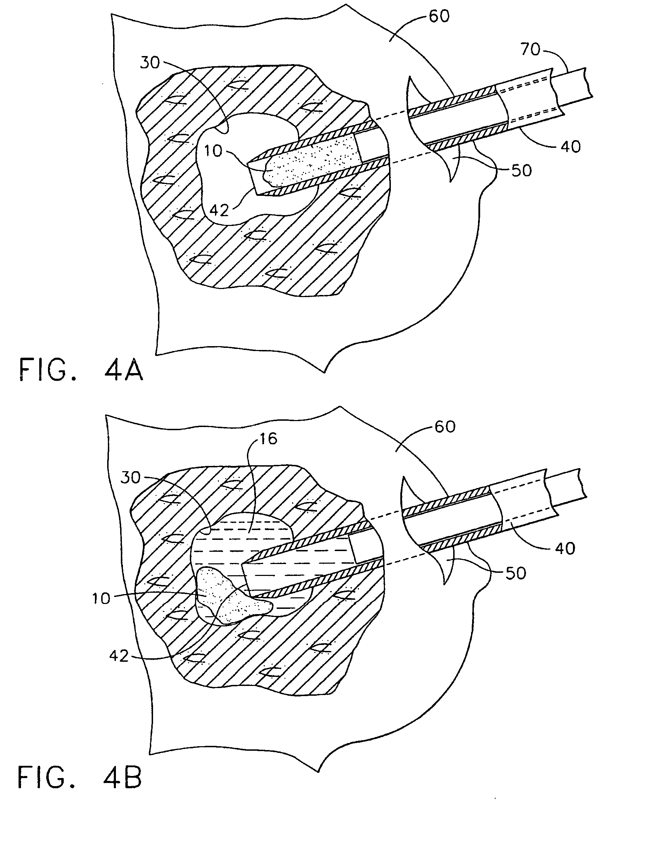

FIG. 4 illustrates (A) a cannula or syringe capable of delivering the tissue cavity marking device into a cavity within the body and (B) as inserted into breast tissue.

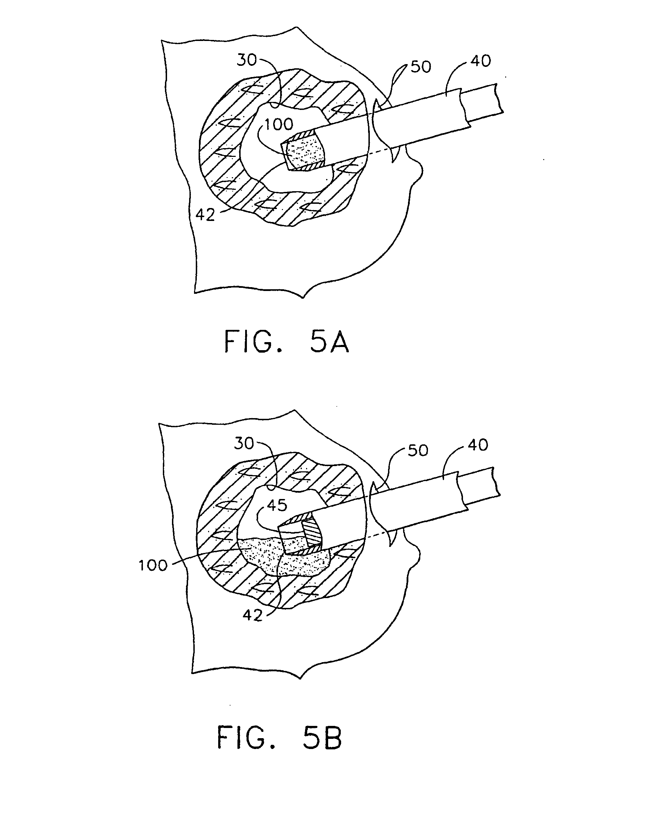

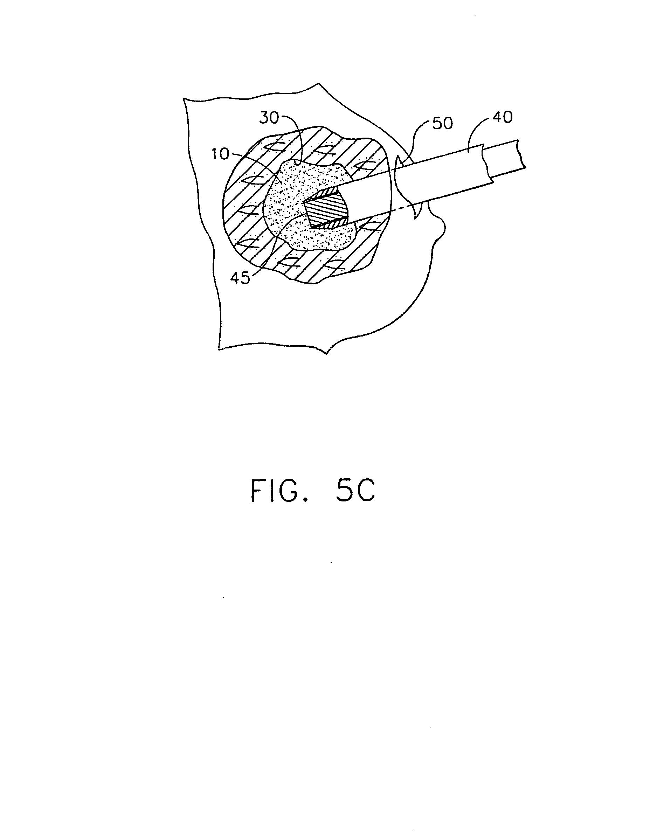

FIG. 5 illustrates (A) a cannula or syringe capable of delivering the tissue cavity marking device into a cavity within the body wherein (A) the marker is placed into the biopsy cavity, (B) the marker swells upon contact with a liquid, and (C) the marker takes on the shape of the biopsy cavity.

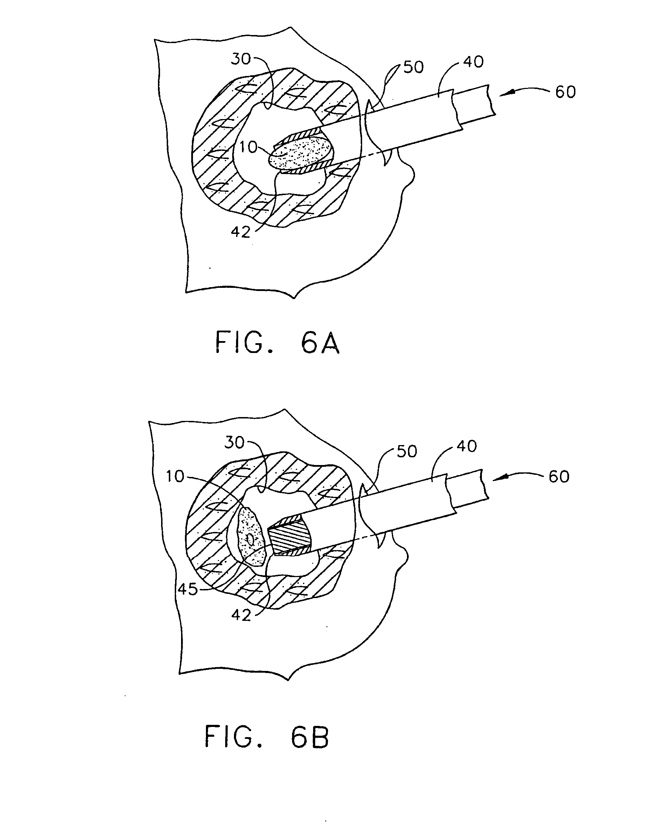

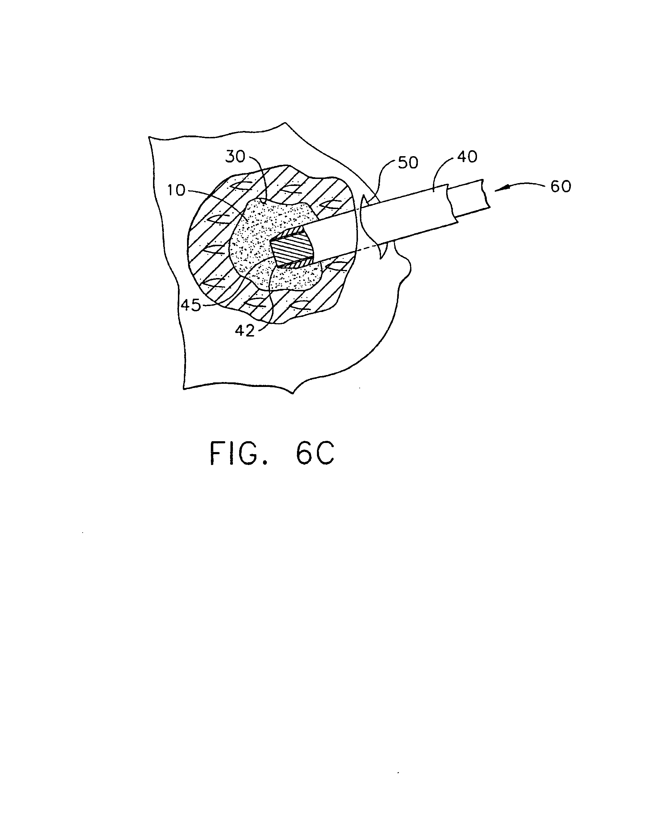

FIG. 6 illustrates (A) a cannula or syringe capable of delivering the tissue cavity marking device encapsulated within a biodegradable shell into a cavity within the body wherein (A) the marker is placed into the biopsy cavity, (B) the biodegradable shell degrades and the marker swells upon contact with a liquid, and (C) the marker takes on the shape of the biopsy cavity.
In the following description of the illustrated embodiments, references are made to the accompanying drawings, which form a part hereof, and in which is shown by way of illustration various embodiments in which the invention may be practiced. It is to be understood that other embodiments may be utilized, and structural and functional changes may be made without departing from the scope of the present invention.

### DETAILED DESCRIPTION OF THE PREFERRED EMBODIMENT

Before the present device and methods for modulation of appetite and satiety are described, it is to be understood that this invention is not limited to the specific methodology, devices. It is also to be understood that the terminology used herein is for the purpose of describing particular embodiments only, and is not intended to limit the scope of the present invention which will be limited only by the appended claims.

It must be noted that as used herein and in the appended claims, the singular forms "a", "and", and "the" include plural referents unless the context clearly dictates otherwise. Thus, for example, reference to "an active agent delivery system" includes a plurality of such devices and reference to "the method of delivery" includes reference to equivalent steps and methods known to those skilled in the art, and so forth.

The invention features devices and methods for making and using a permanent implant marker that is detectable by at least two imaging methods. This invention relates to devices and procedures for percutaneously marking a biopsy cavity. In particular, the inventive device is a biopsy cavity-marking body made of a resilient, substantially permanent material that is MRI, radiopaque and echogenic.

The design parameters of the present invention include that it must remain visible under x-ray and ultrasound, it must not obscure mammograms in future, it cannot look like microcalcifications to be mistaken in future, the ultrasound view should be distinguishable as a marker and not mistaken for a new mass, especially not spiculated, the permanent x-ray visible marker must be obviously manmade so any doctor worldwide would not mistake it for an abnormality or lesion, the material cannot interact with tissue to cause breast to form its own microcalcifications, cannot cause bacterial growth, cannot have negative effect on wound healing, and have minimal to substantially no displacement or migration of the marker.

Preferably, the marker must expand quickly to a size larger than needle tract to prevent popping out a path of least resistance (preferably less than 5 minutes for full expansion). The marker devices of the present invention can be used in variety of cavity sizes- large and small and can be adaptable to a specific deployment means.

In one embodiment, the marking device would be treated with an active agent that is hemostatic, an antibiotic to prevent infections, and or agents that promote healing or tissue growth.

As shown in FIG. 1, this invention relates to devices and procedures for percutaneously marking a biopsy cavity 30. In particular, the inventive device is a biopsy cavity-marking device 100 having a body 10 made of a resilient, preferably non-absorbable polymer material that is radiopaque and echogenic.

This invention further includes the act of filling the biopsy cavity with a nonabsorbable polymer material allowing the material to partially solidify or gel and then placing a marker, which may have a configuration as described above, into the center of the nonabsorbable material.

The permanent, non-erodible polymers that may be used in the present invention include synthetic polymers such as polyacrylates, ethylene-vinyl acetate polymers and other acyl substituted cellulose acetates and derivatives thereof, non-erodible polyurethanes, polystyrenes, polyvinyl chloride, polyvinyl fluoride, poly(vinyl imidazole), chlorosulphonated polyolifins, polyethylene oxide, polyvinyl alcohol, teflon, calcium carbonate, carrageenan and nylon. The preferred non-degradable material for implantation of a marker which is a polyvinyl alcohol gel, foam or sponge, or alkylation, and acylation derivatives thereof, including esters. These materials are all commercially available.

The preferred degradable material for implantation of a marker is where the body of the device is made from a hydrogel selected from the group consisting of a crosslinked polyethylene oxide, polypropylene oxide, polyvinyl alcohol, polyvinyl acetate, polyvinyl pyrrolidone, polyhydroxyalkyl acrylate, polystyrene sulfonate and copolymers or combinations thereof.

The device 100 may take on a variety of shapes and sizes tailored for the specific biopsy cavity 30 within the tissue 60 to be filled. In an alternative embodiment, the inventive device is a biopsy cavity-marking body 10 made of a resilient, non-bioabsorbable material having at least one radiopaque or echogenic marker 12 embedded within the body 10.

A further aspect of the invention allows the marker body 10 to be constructed to have a varying rate of swelling within the body either upon contact with bodily fluids or with an added reactive agent delivered coincidentally.

A further aspect of the invention allows the marker or the body to be constructed to have a layer of bioabsorbable material as an outer "shell." Accordingly, as shown in FIG. 3, the body 10 may be constructed to have a layer of bioabsorbable material as an outer shell 14. Upon degradation of the outer shell 14, the remainder of the polymer body would swell and fill the cavity 30. The body within the shell may be of compressed foam or reactive with body fluids. The marker body may swell to take on a predetermined shape within the cavity 30.

Preferred biodegradable materials include natural and synthetic matrices and foams. More preferred biodegradable materials for use in the device are those which can be processed into polymeric matrices or foams, such as collagen.

Biodegradable materials are particularly suitable in applications where it is desired that natural tissue growth be permitted to completely or partially replace the implanted material over time. Accordingly, biocompatibility is ensured and the natural mechanical parameters of the tissue are substantially restored to those of the pre-damaged condition.

Examples of synthetic biodegradable polymers include, but are not limited to, polylactides (PLA), polyglycolic acids (PGA), poly(lactide-co-glycolides) (PLGA), polycaprolactones (PCL), polycarbonates, polyamides, polyanhydrides, polyamino acids, polyortho esters, polyacetals, polycyanoacrylates, and degradable polyurethanes. Examples of natural biodegradable polymers include, but are not limited to, albumin, collagen, synthetic polyamino acids, prolamines, polysaccharides such as alginate, heparin, and other biodegradable polymers of sugar units.

Examples of natural fibrous biodegradable polymers include, but are not limited to, collagen, elastin, and reticulin. Most preferred as the fibrous material are collagen fibers. Fibrous materials suitable for use in the invention can be prepared by various techniques, such as crosslinking as taught by Stone, U.S. Pat. No. 5,258,043, the entire text of which is incorporated herein by reference. Structural integrity of such polymeric materials can be significantly prolonged by higher average molecular weights of approximately 90,000 daltons or higher, as compared to shorter term degradation molecular weights of approximately 30,000 daltons or less.

The device is usually inserted into the body either surgically via an opening 50 in the body cavity 30, or through a minimally invasive procedure using such devices as a catheter, introducer or similar type insertion device 40. When inserted via the minimally invasive procedure, the resiliency of the body allows the device to be compressed upon placement in a delivery device. Upon insertion of the cavity marking device 100 into the cavity 30, the marker body 10 swells and causes the cavity marking device 100 to self-expand, substantially filling the cavity 30. The resiliency of the body 10 can be further pre-determined so that the body is palpable, thus allowing tactile location by a surgeon in subsequent follow-up examinations.

The device is preferably, although not necessarily, delivered immediately after removal of the tissue specimen using the same device used to remove the tissue specimen itself. Such devices are described in the art. In one embodiment, the device is compressed and loaded into the access device 40 and percutaneously advanced to the biopsy site cavity 30 where, upon exiting from the access device, it expands to substantially fill the cavity 30 of the biopsy. Follow-up noninvasive detection techniques, such as x-ray mammography or ultrasound may then be used by the physician to identify, locate, and monitor the biopsy cavity site over a preferred period of time.

The device 100 may additionally contain a variety of drugs, such as hemostatic agents, pain-killing substances, or even healing or therapeutic agents that may be delivered directly to the biopsy cavity. Importantly, the device is capable of accurately marking a specific location, such as the center, of the biopsy cavity, and providing other information about the patient or the particular biopsy or device deployed.

The expansion of the resilient body 10 can be aided by the addition of a bio-compatible fluid that is absorbed into the body. For instance, the fluid can be a saline solution, a painkilling substance, a healing agent, a therapeutic fluid, or any combination of such fluids. The fluid or combination of fluids may be added to and absorbed by the body 10 of the device 100 before or after deployment of the device into a cavity 30. For example, the body 10 of the device 100 may be pre-soaked with a biocompatible fluid and then delivered into the cavity. In this instance, the fluid aids the expansion of the body of the device 100 upon deployment. Another example is provided as the device is delivered into the cavity without being pre-soaked. In such a case, fluid is delivered into the cavity after the body of the device is deployed into the cavity. Upon delivery of the fluid, the body of the device soaks the fluid, thereby aiding the expansion of the cavity marking device as it expands to fit the cavity. The fluid may be, but is not limited to being, delivered by the access device.

By "bio-compatible fluid" what is meant is a liquid, solution, or suspension that may contain inorganic or organic material. For instance, the bio-compatible fluid is preferably saline solution, but may be water or contain adjuvants such as medications to prevent infection, reduce pain, or the like. Obviously, the liquid is intended to be a type that does no harm to the body.

The body material may also be made radiopaque or echogenic by the addition of radiopaque materials, such as barium- or bismuth-containing compounds and the like, as well as particulate radio-opaque fillers, e.g., powdered tantalum or tungsten, barium carbonate, bismuth oxide, barium sulfate, to the material.

This method may be combined with any aspect of the previously described devices as needed. For instance, one could insert a hemostatic or pain-killing substance as described above into the biopsy cavity along with the nonabsorbable polymer material. Alternatively, a nonabsorbable marker could be inserted into a predetermined location, such as the center, of the body of nonabsorbable material.

This procedure may be used in any internal, preferably soft, tissue, but is most useful in breast tissue, lung tissue, prostate tissue, lymph gland tissue, *etc.* Obviously, though, treatment and diagnosis of breast tissue problems forms the central theme of the invention.

In contrast to the marker clips, the cavity marking device has the obvious advantage of marking the geometric center of a biopsy cavity. Also, unlike the marking clip which has the potential of attaching to loose tissue and moving after initial placement, the marking device self-expands upon insertion into the cavity, thus providing resistance against the walls of the cavity thereby anchoring itself within the cavity. The marking device may be configured to be substantially smaller, larger, or equal to the size of the cavity, however, in some cases the device will be configured to be larger than the cavity. This aspect of the biopsy marking device provides a cosmetic benefit to the patient, especially when the biopsy is taken from the breast. For example, the resistance provided by the cavity marking device against the walls of the cavity may minimize any "dimpling" effect observed in the skin when large pieces of tissue are removed, as, for example, during excisional biopsies.

FIGS. 1-3 show various configurations of a preferred subcutaneous cavity marking device of the present invention. Here the marking device 100 is displayed as having either a generally spherical or cylindrical body. In general, it is within the scope of this invention for the body to assume a variety of shapes. For example, the body may be constructed to have substantially curved surfaces, such as the preferred spherical and cylindrical bodies. Finally, the body may also have an irregular or random shape, in the case of a gel, powder or liquid. The particular body shape will be chosen to best match to the biopsy cavity in which the device is placed. However, it is also contemplated that the body shape can be chosen to be considerably larger than the cavity. Therefore, expansion of the device will provide a significant resistance against the walls of the cavity. Moreover, the aspect ratio of the device is not limited to what is displayed in the figures.

In the bodies of FIGS 2 and 3, marker 12 is located at or near the geometric center of the body 10. Such a configuration will aid the physician in determining the exact location of the biopsy cavity, even after the body swells and fills the cavity. It can be appreciated that any asymmetric marker is useful in aiding a physician to determine the spatial orientation of the deployed inventive device.

The markers 12 herein described may be affixed to the interior or on the surface of the body by any number of suitable methods. For instance, the marker 12 may be merely suspended in the interior of the body 10 (especially in the case where the body is a polymer gel or foam), it may be woven into the body (especially in the case where the marker is a wire or suture), it may be press fit onto the body (especially in the case where the marker is a ring or band), or it may affixed to the body by a biocompatible adhesive. Any suitable means to affix or suspend the marker into the body in the preferred location is within the scope of the present invention.

FIG. 3 depicts a further embodiment of the present invention in which the body 10 is enveloped in a outer shell 14 consisting of a layer of bioabsorbable material such as collagen, cross-linked collagen, regenerated cellulose, synthetic polymers, synthetic proteins, and combinations thereof. Examples of synthetic bioabsorbable polymers that may be used for the body of the device are polyglycolide, or polyglycolic acid (PGA), polylactide, or polylactic acid (PLA), poly ε-caprolactone, polydioxanone, polylactide-co-glycolide, e.g., block or random copolymers of PGA and PLA, and other commercial bioabsorbable medical polymers.

This configuration allows the perimeter of the biopsy cavity to be marked to avoid exposing the cavity, in the case of a margin where re-excision may be necessary, to remaining cancerous cells as the tissue begins to re-grow into the cavity. Such a shell 14 can be radiopaque and/or echogenic in situ, or it may be augmented with an additional coating of an echogenic and/or radiopaque material. The shell 14 can also be made to be palpable so that the physician or patient can be further aided in determining the location and integrity of the implanted inventive device.

The shell 14 may be designed to have a varying bioabsorption rate depending upon the thickness and type of material making up the shell 14. In general, the shell can be designed to degrade over a period ranging from as long as a week or more to as little as several days, hours, or even minutes. It is preferred that such a bioabsorbable shell be designed to degrade between 0.01 and 72 hours; preferred is less than 1 hour, more preferred is less than 5 minutes. In the design of FIG. 3 interior of body 10 may be a swellable, polymer not readily absorbed by the human or mammalian body once the shell 14 degrades. Interior may be filled with a solid or gelatinous material that can be optionally made radiopaque by any number of techniques herein described.

As will be described in additional detail with respect to FIGS. 2-3, marker 12 in the device shown in FIG. 2C and 3C may be permanently radiopaque or echogenic, or it also may be optionally coated with a radiopaque and/or echogenic coating. It is more important from a clinical standpoint that the marker remain detectable either permanently or, if the patient is uncomfortable with such a scenario, for at least a period of about one to five years so that the physician may follow up with the patient to ensure the health of the tissue in the vicinity of the biopsy cavity.

Each of the bodies 10 depicted in FIGS. 1-6 may be made from a wide variety of solid, liquid, aerosol-spray, spongy, or expanding gelatinous nonabsorbable materials such as synthetic polymers.

The device may also be made to emit therapeutic radiation to preferentially treat any suspect tissue remaining in or around the margin of the biopsy cavity. It is envisioned that the marker would be the best vehicle for dispensing such local radiation treatment or similar therapy.

An important aspect of the invention is that the marker is radiopaque and echogenic so that it can be located by non-invasive techniques. Such a feature can be an inherent property of the material used for the marker. Alternatively, a coating or the like can be added to the marker to render the marker detectable or to enhance its detectability. For radiopacity, the marker may be made of a non-bioabsorbable radiopaque material such as platinum, platinum-iridium, platinum-nickel, platinum-tungsten, gold, silver, rhodium, tungsten, tantalum, titanium, nickel, nickel-titanium, their alloys, and stainless steel or any combination of these metals. The material may also be mammographic. By mammographic we mean that the component described is visible under radiography or any other traditional or advanced mammography technique in which breast tissue is imaged.

As previously discussed, the marker can alternatively be made of or coated with a nonabsorbable material. In this case, the marker can, for instance, be made from an additive-loaded polymer. The additive is a radiopaque, echogenic, or other type of substance that allows for the non-invasive detection of the marker. In the case of radiopaque additives, elements such as barium- and bismuth-containing compounds, as well as particulate radio-opaque fillers, *e.g.,* powdered tantalum or tungsten, barium carbonate, bismuth oxide, barium sulfate, *etc.* are preferred. To aid in detection by ultrasound or similar imaging techniques, any component of the device may be combined with an echogenic coating. One such coating is ECHO-COAT from STS Biopolymers. Such coatings contain echogenic features that provide the coated item with an acoustically reflective interface and a large acoustical impedance differential. As stated above, an echogenic coating may be placed over a radiopaque marker to increase the accuracy of locating the marker during ultrasound imaging.

Note that the radiopacity and echogenicity described herein for the marker and the body are not mutually exclusive. It is within the scope of the present invention for the marker or the body to be radiopaque but not necessarily echogenic, and for the marker or the body to be echogenic but not necessarily radiopaque. It is also within the scope of the invention that the marker and the body are both capable of being simultaneously radiopaque and echogenic. For example, if a platinum marker were coated with an echogenic coating, such a marker would be readily visible under x-ray and ultrasonic energy. A similar configuration can be envisioned for the body or for a body coating.

The marker is preferably large enough to be readily visible to the physician under x-ray or ultrasonic viewing, for example, yet be small enough to be able to be percutaneously deployed into the biopsy cavity and to not cause any difficulties with the patient. More specifically, the marker will not be large enough to be palpable or felt by the patient.

Any of the previously-described additional features of the inventive device, such as presence of pain-killing or hemostatic drugs, the capacity for the marker to emit therapeutic radiation for the treatment of various cancers, the various materials that may make up the marker and body, as well as their size, shape, orientation, geometry, *etc*. may be incorporated into the device described above.

Turning now to FIGS.4-6, a method of delivering the inventive device of FIGS. 1-3 is shown. FIG. 5A details the marking device 100 just prior to delivery into a tissue cavity 30 of human or other mammalian tissue, preferably breast tissue 60. As can be seen, the step illustrated in FIG. 5A shows a suitable tubular percutaneous access device 40, such as a catheter or delivery tube, with a distal end 42 disposed in the interior of cavity 30. As previously described, the marking device 100 may be delivered percutaneously through the same access device 40 used to perform the biopsy in which tissue was removed from cavity 30. Although this is not necessary, it is less traumatic to the patient and allows more precise placement of the marking device 100 before fluid begins to fill the cavity 30.

In FIG. 5B marking device 100 is shown being pushed out of the distal end 42 of access device 40 by a pusher or plunger 70 and resiliently expanding to substantially fill the tissue cavity 30.

Finally, in FIG. 5C, access device 40 is withdrawn from the breast tissue, leaving marking device 100 deployed to substantially fill the entire cavity 30 with a marker 12 suspended in the geometric center of the marking device 100 and the cavity 30. As mentioned above, the marking device 100 may be sized to be larger than the cavity 30 thus providing a significant resistance against the walls of the cavity 30.

FIGS. 5A-B and 6A-C show a method of delivering the marking device 100 into a tissue cavity 30 by a plunger 70 that is capable of both advancing the marking device 100 and delivering a bio-compatible fluid 16. The "bio-compatible fluid" is a liquid, solution, or suspension that may contain inorganic or organic material. The fluid is preferably a saline solution, but may be water or contain adjuvants such as medications to prevent infection, reduce pain, or the like. Obviously, the fluid is intended to be a type that does no harm to the body.

FIG. 6A details the marking device 100 prior to delivery into the tissue cavity 30. In FIG. 6B, a plunger 70 pushes the marking device 100 out of the access device 40. Upon exiting the access device 40 the marking device 100 begins resiliently expanding to substantially fill the cavity 30. When the plunger also delivers a bio-compatible fluid 16 into the cavity 30, the fluid aids the marking device 100 in expanding to substantially fill the cavity 30. The bio-compatible fluid may be delivered prior to or subsequent to the placement of the marking device 100 in the cavity 30. The marking device 100 may also be soaked with fluid prior to placement in the cavity 30.

From the foregoing, it is understood that the invention provides an improved subcutaneous cavity marking device and method. While the above descriptions have described the invention for use in the marking of biopsy cavities, the invention is not limited to such. One such application is evident as the invention may further be used as a lumpectomy site marker. In this use, the cavity marking device 100 provides an improved benefit by marking the perimeter of the cavity, e.g., a lumpectomy cavity.

After having been deposited at the biopsy site the marker 100 slowly absorbs moisture from the surrounding tissue and becomes hydrated. In the dehydrated form, shown in the appended drawing figures, the gelatin body or pellet 10 is approximately 1 to 3 mm in diameter and is approximately 5 to 10 mm long. The presently preferred embodiment of the gelatin body 10 is approximately 2 mm in diameter and is approximately 8 mm long. After the body 20 has reached hydration equilibrium with the surrounding tissue it becomes approximately 3 to 5 mm in diameter and approximately 10 to 15 mm long. After hydration the presently preferred embodiment of the body 10 is approximately 4 mm in diameter and approximately 10 mm long.

A visually detectable substance, such as carbon particles, or a suitable dye (e.g. methylene blue or indigo) may also be added to the polymer to make the marker visible by a surgeon during dissection of the surrounding breast tissue.

Materials or compositions which are suitable for the marker 12 include metal, such as stainless steel, tantalum, titanium, gold, platinum, palladium, various alloys that are normally used in bioprosthesis and ceramics and metal oxides that can be compressed into specific shapes or configurations. Among these, the use of biocompatible metals is presently preferred, and the described preferred embodiment of the marker 12 is made of stainless steel. Generally speaking the marker 12 is approximately 0.010 to 0.060 inches wide, approximately 0.030 to 0.200" long and approximately 0.002 to 0.020" thick. The presently preferred permanent marker 22 shown in the drawing figures has the configuration or shape approximating a letter "E", is approximately 0.10" long and approximately 0.040" wide. The letter is readily distinguishable under X-ray and mammography as a "man-made" marker object from any naturally formed X-ray opaque body. Various manufacturing techniques are well known in the art and can be utilized to manufacture the X-ray opaque permanent marker 12. Thus, the marker 12 can be formed from wire, or can be electrochemically etched or laser cut from metal plates.

The marker stays substantially permanently at the biopsy site or is considered permanent.

The drawing figures, particularly FIGS. 1 and 2 show the metal marker 12 disposed substantially in the center of the cylindrical pellet-shaped marking device 100. This is preferred but is not necessary for the present invention. The metal marker 12 can be embodied in or included in the body 10 virtually anywhere. The body 10 however has to have sufficient integrity or firmness to retain the metal marker 12.

A biocompatible liquid or fluid is a liquid that may be introduced into a patient's body without harming the patient. Sterile saline and sterile water containing a sugar (such as dextrose, sucrose or other sugar) or other suitable osmotically-active compounds are typical biocompatible liquids. Other liquids, including fluids not containing water, such as biocompatible oils, may also be used. A biocompatible liquid may be mixed with other agents or materials and used to carry contrast agents, colorants, markers, inert agents, and pharmaceutical agents into a patient.

Pharmaceutical agents, as used herein, are agents used to treat a disease, injury, or medical condition, and include, but are not limited to, drugs, antibiotics, cancer chemotherapy agents, hormones, anesthetic agents, hemostatic agents, and other medicinal compounds. Hemostatic agents are agents that tend to reduce bleeding, enhance clotting, or to cause vasoconstriction in a patient. Brachytherapy agents are typically sources of radiation for implantation near to the site of a cancerous lesion.

Many properties of a marker material affect the intensity of its ultrasound reflection, including density, physical structure, molecular material, and shape. For example, sharp edges, or multiple reflecting surfaces on or within an object differing in density from its surroundings enhances a marker's ability to be detected by ultrasound. Interfaces separating materials of different densities, such as between a solid and a gas, produce strong ultrasound signals.

A typical human breast has a substantial number of features that are visualized with ultrasound. These features all have characteristic signals. Fibrous tissue or ligaments tend to show up as bright streaks, fat seems to appear as a dark gray area, the glandular tissue appears as a mottled medium gray mass. Cancerous lesions typically appear as a darker area with a rough outer edge that has reduced through transmission of the ultrasound energy.

However, due to the large amount of fibrous tissue normally present in a human breast, and due to the presence of ligaments running through the breast, a marker that simply has a bright signal alone will not provide a useful signal that can is readily discernable from the many anatomic features normally present within a human breast. Such markers are typically small, being sized to fit within a syringe or other delivery tube, and so are often not readily distinguishable from natural features of the breast, which include occasional small ultrasound-bright spots. One advantage of the ultrasound-detectable biopsy marker materials of the present invention is that the materials provide an ultrasound signal which can be readily differentiated from anatomic structures within the breast, so that the identification and marking of a biopsy cavity does not require extensive training and experience.

Biopsy site marker materials having features of the present invention may be delivered to a biopsy site in dry form, or in wet form, as in a slurry or suspension. Pressure may be applied to the powder in order to eject it from a storage location, such as a delivery tube. Pressure effective to deliver an ultrasound-detectable marker material having features of the invention includes gas pressure, acoustic pressure, hydraulic pressure, and mechanical pressure.

Mechanical pressure may be delivered by, for example, direct contact with a plunger. A preferred method for delivering an ultrasound-detectable polymer to a biopsy site utilizes a biocompatible liquid to drive or carry the powder into the biopsy cavity at the biopsy site. For example, a quantity of ultrasound-detectable polymer may be contained within a tube or chamber that leads directly or indirectly to a biopsy site. The polymer may be dispensed by the application of hydraulic pressure applied by a syringe containing sterile saline or other suitable liquid.

In a most preferred embodiment, the marker is contained within a tube termed a "delivery tube" or "delivery device" 40. The tube has an outside diameter that is sized to fit within a cannula, such as a Mammotome or SenoCor 360 cannula. For example, a suitable delivery tube has an outside diameter (OD) of about 0.096 inches and has an inner diameter (ID) of about 0.074 inches. Other sizes are also suitable, the exact dimensions depending on the biopsy device used. In addition, a delivery tube may have markings to aid in determining the depth of the tube within a cannula, surface features (such as pins, slots, bumps, bars, wedges, luer-lock fittings, or bands, including a substantially conical circumferential band) effective to control the depth into which a delivery tube is fitted within a cannula or effective to lock a delivery tube into position within a cannula. For example, a delivery tube may have pins or bumps configured to engage a slot or a leading edge of a cannula, or a luer-lock fitting configured to lock into a cannula.

A cannula may also be configured to receive and to engage a delivery tube. A cannula may have pins, slots, wedges, bumps, bands, luer-lock fittings, or the like, to engage a delivery tube and to hold it into a desired position within the cannula. For example, a cannula may have a luer-lock fitting, or a slot to engage a pin on a delivery tube, or an internal bump wedge or band that limits the distance of travel of the delivery tube within the cannula. Delivery tubes embodying features of the present invention may be made of any suitable bio-compatible material.

A fluid 16 used to deposit the marker at a biopsy site may contain other agents, including inert agents, osmotically active agents, pharmaceutical agents, and other bio-active agents. For example, a suitable biocompatible liquid may be selected from the group consisting of sterile saline, sterile saline containing a pharmaceutical agent, sterile saline containing an anesthetic agent, sterile saline containing a hemostatic agent, sterile saline containing a colorant, sterile saline containing a radio contrast agent, sterile sugar solution, sterile sugar solution containing a pharmaceutical agent, sterile sugar solution containing an anesthetic agent, sterile sugar solution containing a hemostatic agent, sterile sugar solution containing a colorant, sterile sugar solution containing a radio contrast agent, biocompatible oils, biocompatible oils containing a pharmaceutical agent, biocompatible oils containing an anesthetic agent, biocompatible oils containing a hemostatic agent, biocompatible oils containing a radio contrast agent, and biocompatible oils containing a colorant. For example, anesthetic agents may be beneficial by reducing patient discomfort.

Hemostatic agents tend to reduce bleeding, enhance clotting, or to cause vasoconstriction in a patient. Hemostatic agents include adrenochrome, algin, alginic acid, aminocaproic acid, batroxobin, carbazochrome salicylate, cephalins, cotarmine, ellagic acid, epinephrine, ethamsylate, factor VIII, factor IX, factor XIII, fibrin, fibrinogen, naphthoquinone, oxamarin, oxidized cellulose, styptic collodion, sulamrin, thrombin, thromboplastin (factor III), tolonium chloride, tranexamic acid, and vasopression.

Pharmaceutical agents are often used to promote healing, and to treat injury, infection, and diseases such as cancer, and may include hormones, hemostatic agents and anesthetics as well as antibacterial, antiviral, antifungal, anticancer, and other medicinal agents. Pharmaceutical agents may be included as part of an ultrasound-detectable bioresorbable material placed within a biopsy cavity in order, for example, to promote healing, prevent infection, and to help treat any cancer cells remaining near the biopsy site.

### Additives to Polymer Markers

In some embodiments it may be desirable to add bioactive molecules to the markers. A variety of bioactive molecules can be delivered using the matrices described herein. These are referred to generically herein as "factors" or "bioactive factors".

In the preferred embodiment, the bioactive factors are growth factors, angiogenic factors, compounds selectively inhibiting in-growth of fibroblast tissue such as anti-inflammatories, and compounds selectively inhibiting growth and proliferation of transformed (cancerous) cells. These factors may be utilized to control the growth and function of implanted cells, the in-growth of blood vessels into the forming tissue, and/or the deposition and organization of fibrous tissue around the implant.

Examples of growth factors include heparin binding growth factor (HBGF), transforming growth factor alpha or beta (TGF-beta), alpha fibroblastic growth factor (FGF), epidermal growth factor (TGF), vascular endothelium growth factor (VEGF), some of which are also angiogenic factors. Other factors include hormones such as insulin, glucagon, and estrogen. In some embodiments it may be desirable to incorporate factors such as nerve growth factor (NGF) or muscle morphogenic factor (MMP).

Steroidal anti-inflammatories can be used to decrease inflammation to the implanted marker, thereby decreasing the amount of fibroblast tissue growing into the marker.

These factors are known to those skilled in the art and are available commercially or described in the literature. Preferably, the bioactive factors are incorporated to between one and 30% by weight, although the factors can be incorporated to a weight percentage between 0.01 and 95 weight percentage.

Bioactive molecules can be incorporated into the marker and released over time by diffusion and/or degradation of the marker, they can be incorporated into microspheres which are attached to or incorporated within the marker, or some combination thereof.

### Polymer Solutions

Polymeric materials that are capable of forming a hydrogel may be utilized. The polymer is mixed with cells for implantation into the body and is permitted to crosslink to form a hydrogel marker containing the cells either before or after implantation in the body. In one embodiment, the polymer forms a hydrogel within the body upon contact with a crosslinking agent. A hydrogel is defined as a substance formed when an organic polymer (natural or synthetic) is crosslinked via covalent, ionic, or hydrogen bonds to create a three-dimensional open-lattice structure that entraps water molecules to form a gel. Naturally occurring and synthetic hydrogel forming polymers, polymer mixtures and copolymers may be utilized as hydrogel precursors.

Examples of materials that can be used to form a hydrogel include modified alginates. Alginate is a carbohydrate polymer isolated from seaweed, which can be crosslinked to form a hydrogel by exposure to a divalent cation such as calcium, as described. The modified alginate solution is mixed with the cells to be implanted to form a suspension. Then the suspension is injected directly into a patient prior to crosslinking of the polymer to form the hydrogel containing the cells. The suspension then forms a hydrogel over a short period of time due to the presence in vivo of physiological concentrations of calcium ions.

Alginate is ionically cross-linked in the presence of divalent cations, in water, at room temperature, to form a hydrogel marker. Due to these mild conditions, alginate has been the most commonly used polymer for hybridoma cell encapsulation, as described, for example, in U.S. Pat. No. 4,352,883 to Lim. In the Lim process, an aqueous solution containing the biological materials to be encapsulated is suspended in a solution of a water soluble polymer, the suspension is formed into droplets which are configured into discrete microcapsules by contact with multivalent cations, then the surface of the microcapsules is cross-linked with polyamino acids to form a semipermeable membrane around the encapsulated materials.

Other polymeric hydrogel precursors include polyethylene oxide-polypropylene glycol block copolymers such as Pluronics or Tetronics, which are cross-linked by hydrogen bonding and/or by a temperature change, as described in Steinleitner *et al.,* Obstetrics & Gynecology, 77:48-52 (1991); and Steinleitner *et al.,* Fertility and Sterility, 57:305-308 (1992). Other materials that may be utilized include proteins such as fibrin, collagen and gelatin. Polymer mixtures also may be utilized. For example, a mixture of polyethylene oxide and polyacrylic acid which gels by hydrogen bonding upon mixing may be utilized. In one embodiment, a mixture of a 5% w/w solution of polyacrylic acid with a 5% w/w polyethylene oxide (polyethylene glycol, polyoxyethylene) 100,000 can be combined to form a gel over the course of time, e.g., as quickly as within a few seconds.

Covalently cross-linkable hydrogel precursors also are useful. For example, a water-soluble polyamine, such as chitosan, can be cross-linked with a water-soluble diisothiocyanate, such as polyethylene glycol diisothiocyanate. The isothiocyanates will react with the amines to form a chemically cross-linked gel. Aldehyde reactions with amines, e.g., with polyethylene glycol dialdehyde also may be utilized. A hydroxylated water-soluble polymer also may be utilized.

Alternatively, polymers may be utilized which include substituents that are cross-linked by a radical reaction upon contact with a radical initiator. For example, polymers including ethylenically unsaturated groups that can be photochemically cross-linked may be utilized. In this embodiment, water-soluble macromers that include at least one water-soluble region, a biodegradable region, and at least two free radical-polymerizable regions, are provided. The macromers are polymerized by exposure of the polymerizable regions to free radicals generated, for example, by photosensitive chemicals and or light. Examples of these macromers are PEG-oligolactyl-acrylates, wherein the acrylate groups are polymerized using radical initiating systems, such as an eosin dye, or by brief exposure to ultraviolet or visible light. Additionally, water-soluble polymers, which include cinnamoyl groups, which may be photochemically cross-linked, may be utilized.

In general, the polymers are at least partially soluble in aqueous solutions, such as water, buffered salt solutions, or aqueous alcohol solutions. Methods for the synthesis of the other polymers described above are known to those skilled in the art. See, for example Concise Encyclopedia of Polymer Science and Polymeric Amines and Ammonium Salts, E. Goethals, editor (Pergamen Press, Elmsford, N.Y. 1980). Many polymers, such as poly(acrylic acid), are commercially available. Naturally occurring and synthetic polymers may be modified using chemical reactions available in the art.

Water soluble polymers with charged side groups may be cross-linked by reacting the polymer with an aqueous solution containing ions of the opposite charge, either cations if the polymer has acidic side groups or anions if the polymer has basic side groups. Examples of cations for crosslinking of the polymers with acidic side groups to form a hydrogel are monovalent cations such as sodium, and multivalent cations such as copper, calcium, aluminum, magnesium, strontium, barium, and tin, and di-, tri- or tetra-functional organic cations such as alkylammonium salts. Aqueous solutions of the salts of these cations are added to the polymers to form soft, highly swollen hydrogels and membranes. The higher the concentration of cation, or the higher the valence, the greater the degree of cross-linking of the polymer. Additionally, the polymers may be cross-linked enzymatically, e.g., fibrin with thrombin.

The device is provided in a configuration sufficient to provide a secure, compliant fit, given the dimensions of the biological aperture within which it is placed. An optimal configuration (e.g. shape and dimensions) of the device can be determined based upon the natural and/or desired geometry of the implant site. The device configuration can be modified according to particular dimensions of the aperture and/or particular desired functional requirements.

Numerous techniques (e.g., selection, shaping, sculpting or molding techniques) can be used in order to configure a device for a particular implant site, depending largely upon the aperture itself and the particular material used. When porous polyvinyl alcohol (PVA) is used as the material for the device, suitable techniques include cutting and heat molding. Accordingly, the porous PVA material can be cut or carved using a knife or scissors. Porous PVA also tends to exhibit thermoplastic properties when saturated with water, which enables the material to be configured by heat molding it into various dimensions and shapes upon heating and/or drying. A preferred heat molding process for porous PVA generally includes the steps of a) wetting the material, b) cutting or sculpting the wetted material to a shape and dimension which is between about 10% and about 30% larger than that of the desired final form (when heat treated, porous PVA tends to permanently lose approximately 20% of its original size), c) encasing the material into a mold having the desired configuration, d) immersing the mold-encased material into boiling water or steam, e) subsequently cooling the mold-encased material, and finally, f) removing the material from the mold.

The device of the invention can be provided having a configuration that best accommodates the dimensions of an aperture into which it is positioned, such as an annular herniation or access aperture. It is generally desirable for the device to fit securely in, as well as penetrate into, and preferably through, the annulus to the extent desired. The normal height of an intact annulus at its periphery is approximately one centimeter, although this varies according to the individual patient and tends to become smaller with age and can be affected by injury or compressive damage to the disc.

Alternatively, the device can contain an elongated configuration adapted for a dual aperture system (not shown) in which each end of the elongated device resides in respective apertures, typically on opposing sides of the annulus. This dual aperture system permits the practitioner to surgically maneuver both ends of the device into the proper position during surgery.

Optionally, or in addition to porosity, one or more bioactive agents can be incorporated into the device, e.g., onto or into the marker material itself and/or one or more other materials making up the device. Bioactive agents suitable for use include natural and synthetic compounds, examples of which are bioactive polypeptides, proteins, cells, and the like, which permit (*e.g*., stimulate) tissue ingrowth. Preferred bioactive agents are those which actively facilitate tissue ingrowth and/or improve the biocompatibility of the device when used in conjunction with the material of the device.

Suitable bioactive agents include, but are not limited to, tissue growth enhancing substances such as growth factors, angiogenic factors, immune system suppressors such as anti-inflammatory agents, antibiotics, living cells, cell-binding proteins and peptides, and the like. Growth factors that enhance cartilage repair are particularly preferred for use as bioactive agents. Examples of suitable growth factors are selected from the group consisting of somatomedins (somatomedin-C), insulin-like growth factors (such as IGF-I and II), fibroblast growth factors (including acidic and basic FGF), bone morphogenic factors (e.g., BMP and BMP2), endothelial cell growth factors, transforming growth factors (TGF alpha and beta), platelet derived growth factors ("PDGF"), hepatocytic growth factors, keratinocyte growth factors, and combinations thereof. Growth factors that function by attracting fibroblasts are preferred, as are growth factors that encourage fibroblast growth, either directly or indirectly by encouraging mesenchymal cell development.

The bioactive agent can be either immobilized upon the implanted device and/or it can be released therefrom in situ. Growth factors can be incorporated in a releasable fashion using conventional controlled release methods, including but not limited to encapsulation or microspheres. Selection of the particular bioactive agent(s) for use with the device and the controlled release technique thereof will vary, as those skilled in the art will appreciate, according to the particular implant tissue site.

A device can be formed of a single material throughout (which itself is either homogeneous throughout or having regions of varying chemical/physical properties), or it can be formed of one or more materials (e.g., in temporary or permanent attached or touching contact), each having different physical and/or chemical properties. In one embodiment as depicted in FIG. __, the device 100 includes discrete internal and external portions 20 and 21, respectively, wherein the internal portion 10 is provided in the form of a semi-rigid material used to provide mechanical support. Either or both portions can contain bioactive agents and/or other substances, e.g., to enable device imaging such as radio-opaque materials, to provide varied hydration expansion rates, and the like. Materials useful as the internal portion 10 can include polymers, such as polyethylene, and metals, such as titanium or stainless steel, for example. Composite materials can be used as well, namely composites comprising polymeric foams on the external surface. Materials that permit imaging by MRI or X-ray, for example, can be used as well, offering the advantage of being able to monitor the migration of the device both during surgery and over the long-term.

Examples of materials useful as additional components of the device include, but are not limited to, polymers, plastics, inert metals such as stainless steel, aluminum, titanium, palladium, metallic alloys, insoluble inert metal oxides, phosphates, silicates, carbides, silicon carbide, carbon, ceramics or glass, polycarbonate, polystyrene, epoxyresins, silicone, cellulose acetate, cellulose nitrate, cellophane, PTFE (Teflon), polyethylene terephthalate, polyformaldehyde, fluorinated ethylenepropylene co-polymer, polyphenylene oxide, polypropylene, mica, collagen, and the like.

The material used in the device of the invention can be either expandable or nonexpandable. Preferably, whatever portion or portions of the device are adapted to directly contact the surrounding tissue implantation site are expandable in situ upon implantation. In their contracted (or unexpanded) form, preferred materials can be adapted for substantially minimally invasive introduction to the tissue site, where upon expansion, they serve to secure the device in place and provide immediate structural support. Upon expansion, a preferred device offers the advantage of being compliant with the aperture, that is, able to substantially conform itself to the shape and dimensions of the aperture, including any irregularities or aberrations in the aperture or implant site. In a related embodiment, a device can be adapted to provide, or tend toward, a predetermined shape and dimensions in its expanded form, thereby serving to determine the corresponding shape of the surrounding tissue.

Preferably, the device is secured within the cavity 30, at least temporarily, by expansion of the material in situ, e.g., upon hydration or release from constraining means, e.g., a shell. The extent and kinetics of expansion can be controlled using various methods. For example, hydration expansion of a porous device can be controlled by the use of high molecular weight cross-linking, mechanical compression, chemical additives or coatings, heat treatment, *etc*. Given the present disclosure, those skilled in the art can select the appropriate method or treatment for controlling expansion according to the particular material used.

In addition to selecting specific trocar-type drill diameters, computer programs and other measurement techniques known to one of ordinary skill in the art can be used to aid the practitioner in assessing the dimensions of the prepared tissue site so as to properly configure the device.

The number and configuration of expandable devices used can be varied or altered according to the specific patient's needs. In order to optimize the compliancy of the device to the tissue site, the tissue aperture can itself be adjusted and/or the device can be molded or sculpted according to the aperture. Accordingly, if the damage to the annulus, for example, is more severe, expanding the width of the device to conform to the wider opening in the annulus may be desirable.

The device is preferably sterilized in the course of its manufacture and packaging, or prior to implantation. In the case of a porous PVA having thermoplastic properties, heat treatments or chemical methods of sterilization can sterilize the device.

The invention also relates to methods of initiating formation of hydrogels in situ to form a hydrogel medical device. The invention also relates to initiator systems for initiating formation of hydrogels in situ to form a hydrogel medical device.

Generally, the hydrogel materials appropriate for use in the present invention should be physiologically acceptable and should be swollen in the presence of water. These characteristics allow the hydrogels to be introduced into the body in a "substantially deswollen" state and over a period of time hydrate to fill a void, a defect in tissue, or create a hydrogel-filled void within a tissue or organ by mechanically exerting a gentle force during expansion. The hydrogel may be preformed or formed in situ.

"Substantially deswollen" is defined as the state of a hydrogel wherein an increase in volume of the hydrogel of the article or device formed by such hydrogel is expected on introduction into the physiological environment. Thus, the hydrogel may be in a dry state, or less than equilibrium hydrated state, or may be partially swollen with a pharmaceutically acceptable fluid that is easily dispersed or is soluble in the physiological environment. The expansion process also may cause the implanted material to become firmly lodged within a hole, an incision, a puncture, or any defect in tissue which may be congenital, diseased, or iatrogenic in origin, occlude a tubular or hollow organ, or support or augment tissue or organs for some therapeutic purpose.

Hydrogels useful in practicing the present invention may be formed from natural, synthetic, or biosynthetic polymers. Natural polymers may include glycosminoglycans, polysaccharides, proteins *etc.* The term "glycosaminoglycan" is intended to encompass complex polysaccharides which are not biologically active (i.e., not compounds such as ligands or proteins) and have repeating units of either the same saccharide subunit or two different saccharide subunits. Some examples of glycosaminoglycans include dermatan sulfate, hyaluronic acid, the chondroitin sulfates, chitin, heparin, keratin sulfate, keratosulfate, and derivatives thereof.

In general, the glycosaminoglycans are extracted from a natural source and purified and derivatized. However, they also may be synthetically produced or synthesized by modified microorganisms such as bacteria. These materials may be modified synthetically from a naturally soluble state to a partially soluble or water swellable or hydrogel state. This modification may be accomplished by various well-known techniques, such as by conjugation or replacement of ionizable or hydrogen bondable functional groups such as carboxyl and/or hydroxyl or amine groups with other more hydrophobic groups.

For example, carboxyl groups on hyaluronic acid may be esterified by alcohols to decrease the solubility of the hyaluronic acid. Such processes are used by various manufacturers of hyaluronic acid products (such as Genzyme Corp., Cambridge, Mass.) to create hyaluronic acid based sheets, fibers, and fabrics that form hydrogels. Other natural polysaccharides, such as carboxymethyl cellulose or oxidized regenerated cellulose, natural gum, agar, agarose, sodium alginate, carrageenan, fucoidan, furcellaran, laminaran, hypnea, eucheuma, gum arabic, gum ghatti, gum karaya, gum tragacanth, locust beam gum, arbinoglactan, pectin, amylopectin, gelatin, hydrophilic colloids such as carboxymethyl cellulose gum or alginate gum cross-linked with a polyol such as propylene glycol, and the like, also form hydrogels upon contact with aqueous surroundings.

Synthetic polymeric hydrogels generally swell or expand to a very high degree, usually exhibiting a 2 to 100-fold volume increase upon hydration from a substantially dry or dehydrated state. Synthetic hydrogels may be biostable or biodegradable or bioabsorbable. Biostable hydrophilic polymeric materials that form hydrogels useful for practicing the present invention include poly(hydroxyalkyl methacrylate), poly(electrolyte complexes), poly(vinylacetate) cross-linked with hydrolysable bonds, and water-swellable N-vinyl lactams.

The hydrogel can be any of a number of types that are biocompatible and that form in response to an initiator. The hydrogel is formed from a composition including polymers or macromers that are curable, meaning that they can be cured or otherwise modified, in situ, at the tissue site, in response to an initiator, and undergo a phase or chemical change sufficient to retain a desired position and configuration. Examples include hydrogels formed from macromers, as described in WO 01/68720 to BioCure, Inc. and U.S. Pat. No. 5,410,016 to Hubbell *et al.* The term "gellable composition" is used herein to refer to the polymeric or macromenc composition that forms the hydrogel in response to initiation.

Other suitable hydrogels include hydrophilic hydrogels know as CARBOPOL, a registered trademark of B. F. Goodrich Co., Akron, Ohio, for acidic carboxy polymer (Carbomer resins are high molecular weight, allylpentaerythritol-crosslinked, acrylic acid-based polymers, modified with C10-C30 alkyl acrylates), polyacrylamides marketed under the CYANAMER name, a registered trademark of Cytec Technology Corp., Wilmington, Del., polyacrylic acid marketed under the GOOD-RITE name, a registered trademark of B. F. Goodrich Co., Akron, Ohio, polyethylene oxide, starch graft copolymers, acrylate polymer marketed under the AQUA-KEEP name, a registered trademark of Sumitomo Seika Chemicals Co., Japan, ester cross-linked polyglucan, and the like. Such hydrogels are described, for example, in U.S. Pat. No. 3,640,741 to Etes, U.S. Pat. No. 3,865,108 to Hartop, U.S. Pat. No. 3,992,562 to Denzinger *et al.,* U.S. Pat. No. 4,002,173 to Manning *et al.,* U.S. Pat. No. 4,014,335 to Arnold and U.S. Pat. No. 4,207,893 to Michaels, all of which are incorporated herein by reference.

A gellable composition, formed from the polymers or macromers, and optionally other components, is deliverable to the intended site of application. The properties, i.e. viscosity, of this composition will vary depending upon the intended final use of the composition. For example, a composition intended for use as an embolic device will have certain desired characteristics. The composition is delivered to the intended site through an appropriate delivery device, such as a catheter or syringe. Before, during, or after delivery, the composition is exposed to the initiator system, causing gellation of the polymers or macromers and formation of the hydrogel device.

Gellation of the polymers or macromers can be via a number of mechanisms, such as physical crosslinking or chemical crosslinking. Physical crosslinking includes, but is not limited to, complexation, hydrogen bonding, desolvation, Van der waals interactions, and ionic bonding. Chemical crosslinking can be accomplished by a number of means including, but not limited to, chain reaction (addition) polymerization, step reaction (condensation) polymerization and other methods of increasing the molecular weight of polymers/oligomers to very high molecular weights. Chain reaction polymerization includes, but is not limited to, free radical polymerization (thermal, photo, redox, atom transfer polymerization, *etc.*), cationic polymerization (including onium), anionic polymerization (including group transfer polymerization), certain types of coordination polymerization, certain types of ring opening and metathesis polymerizations, *etc.* Step reaction polymerizations include all polymerizations which follow step growth kinetics including but not limited to reactions of nucleophiles with electrophiles, certain types of coordination polymerization, certain types of ring opening and metathesis polymerizations, *etc.* Other methods of increasing molecular weight of polymers/oligomers include but are not limited to polyelectrolyte formation, grafting, ionic crosslinking, *etc.*

Other means for gellation also may be advantageously used with macromers that contain groups that demonstrate activity towards functional groups such as amines, imines, thiols, carboxyls, isocyanates, urethanes, amides, thiocyanates, hydroxyls, *etc.*

Desirable crosslinkable groups include (meth)acrylamide, (meth)acrylate, styryl, vinyl ester, vinyl ketone, vinyl ethers, *etc.* Particularly desirable are ethylenically unsaturated functional groups.

The hydrogel can be formed from one or more macromers that include a hydrophilic or water-soluble region and one or more cross-linkable regions. The macromers may also include other elements such as one or more degradable or biodegradable regions. A variety of factors--primarily the desired characteristics of the formed hydrogel--determines the most appropriate macromers to use. Many macromer systems that form biocompatible hydrogels can be used.

Macromers suitable for use in the compositions described herein are disclosed in WO 01/68720 to BioCure, Inc. Other suitable macromers include those disclosed in U.S. Pat. Nos. 5,410,016 to Hubbell *et al.,* 4,938,763 to Dunn *et al.,* 5,100,992 and 4,826,945 to Cohn *et al.,* 4,741,872 and 5,160,745 to De Luca et al, and 4,511,478 to Nowinski *et al.*

Devices can be constructed from a number of hydrophilic polymers, such as, but not limited to, polyvinyl alcohols (PVA), polyethylene glycols (PEG), polyvinyl pyrrolidone (PVP), polyalkyl hydroxy acrylates and methacrylates (e.g. hydroxyethyl methacrylate (HEMA), hydroxybutyl methacrylate (HBMA), and dimethylaminoethyl methacrylate (DMEMA)), polysaccharides (e.g. cellulose, dextran), polyacrylic acid, polyamino acids (e.g. polylysine, polyethyimine, PAMAM dendrimers), polyacrylamides (e.g. polydimethylacrylamid-co-HEMA, polydimethylacrylamid-co-HBMA, polydimethylacrylamid-co-DMEMA).

In one preferred embodiment, the device is a polymer comprising units having a 1,2-diol or 1,3-diol structure, such as polyhydroxy polymers. For example, polyvinyl alcohol (PVA) or copolymers of vinyl alcohol contain a 1,3-diol skeleton. The backbone can also contain hydroxyl groups in the form of 1,2-glycols, such as copolymer units of 1,2-dihydroxyethylene. These can be obtained, for example, by alkaline hydrolysis of vinyl acetate-vinylene carbonate copolymers. Other polymeric diols can be used, such as saccharides.

In addition, the macromers can also contain small proportions, for example, up to 20%, preferably up to 5%, of comonomer units of ethylene, propylene, acrylamide, methacrylamide, dimethacrylamide, hydroxyethyl methacrylate, alkyl methacrylates, alkyl methacrylates which are substituted by hydrophilic groups, such as hydroxyl, carboxyl or amino groups, methyl acrylate, ethyl acrylate, vinylpyrrolidone, hydroxyethyl acrylate, allyl alcohol, styrene, polyalkylene glycols, or similar comonomers usually used.

It is also possible to use copolymers of hydrolyzed or partially hydrolyzed vinyl acetate, which are obtainable, for example, as hydrolyzed ethylene-vinyl acetate (EVA), or vinyl chloride-vinyl acetate, N-vinylpyrrolidone-vinyl acetate, and maleic anhydride-vinyl acetate.

Polyvinyl alcohols that can be derivatized as described herein preferably have a molecular weight of at least about 2,000. As an upper limit, the PVA may have a molecular weight of up to 1,000,000. Preferably, the PVA has a molecular weight of up to 300,000, especially up to approximately 130,000, and especially preferably up to approximately 60,000.

The PVA usually has a poly(2-hydroxy)ethylene structure. The PVA derivatized in accordance with the disclosure may, however, also comprise hydroxy groups in the form of 1,2-glycols.

The PVA system can be a fully hydrolyzed PVA, with all repeating groups being --CH_{Z}--CH(OH), or a partially hydrolyzed PVA with varying proportions (1% to 25%) of pendant ester groups. PVA with pendant ester groups have repeating groups of the structure CH₂--CH(OR) where R is COCH₃ group or longer alkyls, as long as the water solubility of the PVA is preserved. The ester groups can also be substituted by acetaldehyde or butyraldehyde acetals that impart a certain degree of hydrophobicity and strength to the PVA. For an application that requires an oxidatively stable PVA, the commercially available PVA can be broken down by NaIO₄--KMnO₄ oxidation to yield a small molecular weight (2000 to 4000) PVA.

The PVA is prepared by basic or acidic, partial or virtually complete hydrolysis of polyvinyl acetate. In a preferred embodiment, the PVA comprises less than 50% of vinyl acetate units, especially less than about 25% of vinyl acetate units. Preferred amounts of residual acetate units in the PVA, based on the sum of vinyl alcohol units and acetate units, are approximately from 3 to 25%.

The term "initiator" is used herein to refer to an element, which begins the process of gelation of a gellable composition. In some cases, the term "initiator" as used herein refers to one part of an initiator system. For example, a redox couple may be used as the initiator system, wherein one part of the couple is included in the gellable composition and the other part of the couple is separately provided. The part of the couple separately provided is referred to as the "initiator" herein.

In one embodiment of the invention, the initiator is provided at the site in the form of a solid article. Examples of solid articles that can be or provide the initiator are microspheres, disks, coils, and other shaped articles. The solid article can be made of metal, such as a metallic coil, or a polymer, such as polymeric microspheres.

There are many ways in which the solid article can embody the initiator. For example, the article can be made entirely or partially of the initiator, the initiator can be coated on the surface of the article, or the initiator can be embedded or impregnated into the article. For example, the solid article could be microspheres or a solid disk made from an initiator. The initiator can be released from the solid article, or simply contact with the solid article can provide initiation.

The solid article initiator is delivered to the site where the hydrogel article is to be formed. It can be delivered before, during, or after the gellable composition is delivered. As one example, the solid initiator could be an embolic coil coated with initiator that is placed in an aneurysm prior to delivery of gellable prepolymer to the aneurysm. The initiator could be microspheres impregnated with an initiator compound that are injected to a site to be bulked after the gellable composition has been delivered to the site. As another example, the initiator could be a polymer sheet coated with initiator compound that is applied to an area to be sealed, prior to application of the gellable composition to the area.

In a case where crosslinkable groups are initiated by free radical polymerization, one part of a redox couple can be delivered along with the macromer solution through a single lumen catheter and the other part of the redox couple can be delivered through the solid article(s). Other types of initiators can also be supplied via a solid article, such as divalent cationic ions for ionic crosslinking of polysaccharides.

In another embodiment of the invention, the initiator is provided as an infusion of a solution containing the initiator. The infusion solution can be provided via a separate access point, or can be provided via the same access point, but downstream of the gellable composition. For example, in the case of embolic agent delivery to a neurovascular aneurysm, the gellable composition can be delivered via a catheter introduced via the femoral artery, as is standard in practice, while the initiator infusion solution can be delivered via a catheter introduced via the carotid artery. In another embodiment, a dual lumen catheter can be employed wherein one lumen extends further than the other so that the catheter diameter is narrower at its distal end (and can access smaller vasculature). The lumens can be arranged coaxially or side by side. The gellable composition is delivered via the shorter lumen, while the initiator infusion solution is delivered via the longer lumen. If the lumens are arranged coaxially, the longer lumen is the internal lumen.

In the example of macromers crosslinked via free radical chemistry using a redox initiator, the macromer solution containing one part of the redox couple can be delivered through a catheter introduced via the femoral artery and the other part of the redox couple can be delivered through a catheter introduced via the carotid artery.

In another embodiment, the initiator system is provided at the delivery tip of the catheter. For example, the catheter tip could provide (deliver) one part of a redox couple while the other part of the couple is provided in a solution of the gellable composition.

It may be desirable to include a contrast agent in the compositions. A contrast agent is a biocompatible (non-toxic) material capable of being monitored by, for example, radiography. The contrast agent can be water soluble or water insoluble. Examples of water soluble contrast agents include metrizamide, iopamidol, iothalamate sodium, iodomide sodium, and meglumine. Iodinated liquid contrast agents include Omnipaque, Visipaque, and Hypaque-76. Examples of water insoluble contrast agents are tantalum, tantalum oxide, barium sulfate, gold, tungsten, and platinum. These are commonly available as particles preferably having a size of about 10 µm or less.

The contrast agent can be added to the compositions prior to administration. Both solid and liquid contrast agents can be simply mixed with a solution of the compositions. Liquid contrast agent can be mixed at a concentration of about 10 to 80 volume percent, more desirably about 20 to 50 volume percent. Solid contrast agents are desirably added in an amount of about 10 to 40 weight percent, more preferably about 20 to 40 weight percent.

It may be desirable to use the compositions in combination with one or more occlusive devices. Such devices include balloons, microcoils, and other devices known to those skilled in the art. The device can be placed at the site to be occluded or filled before, during, or after the composition is administered. For example, an occlusive coil can be placed in an aneurysm sac to be-filled and the liquid composition can be injected into the sac to fill the space around the coil. An advantage of using an occlusive device along with the composition is that it may provide greater rigidity to the filling.

An effective amount of one or more biologically active agents can be included in the compositions. It may be desirable to deliver the active agent from the formed hydrogel. Biologically active agents that it may be desirable to deliver include prophylactic, therapeutic, and diagnostic agents including organic and inorganic molecules and cells (collectively referred to herein as an "active agent" or "drug"). A wide variety of active agents can be incorporated into the hydrogel. Release of the incorporated additive from the hydrogel is achieved by diffusion of the agent from the hydrogel, degradation of the hydrogel, and/or degradation of a chemical link coupling the agent to the polymer. In this context, an "effective amount" refers to the amount of active agent required to obtain the desired effect.

Examples of active agents that can be incorporated include, but are not limited to, anti-angiogenic agents, chemotherapeutic agents, radiation delivery devices, such as radioactive seeds for brachytherapy, and gene therapy compositions.

Chemotherapeutic agents that can be incorporated include water soluble chemotherapeutic agents, such as cisplatin (platinol), doxorubicin (adriamycin, rubex), or mitomycin C (mutamycin). Other chemotherapeutic agents include iodinated fatty acid ethyl esters of poppy seed oil, such as lipiodol.

Active agents can be incorporated into the compositions simply by mixing the agent with the composition prior to administration. The active agent will then be entrapped in the hydrogel that is formed upon administration of the composition. The active agent can be in compound form or can be in the form of degradable or nondegradable nano- or microspheres. It some cases, it may be possible and desirable to attach the active agent to the macromer. The active agent may be released from the macromer or hydrogel over time or in response to an environmental condition.

It may be desirable to include a peroxide stabilizer in redox initiated systems. Examples of peroxide stabilizers are Dequest products from Solutia Inc., such as for example Dequest 2010 and Dequest 2060S. These are phosphonates and chelants that offer stabilization of peroxide systems. Dequest 2060S is diethylenetriamine penta(methylene phosphonic acid). These can be added in amounts as recommended by the manufacturer.

It may be desirable to include fillers in the compositions, such as fillers that leach out of the formed hydrogel over a period of time and cause the hydrogel to become porous. Such may be desirable, for example, where the composition is used for chemoembolization and it may be desirable to administer a follow up dose of chemoactive agent. Appropriate fillers include calcium salts, for example.

The compositions are highly versatile. A number of characteristics can be easily modified, making the compositions suitable for a number of applications. For example, as discussed above, the polymer backbones can include co-monomers to add desired properties, such as, for example, thermoresponsiveness, degradability, gelation speed, and hydrophobicity. Modifiers can be attached to the polymer backbone (or to pendant groups) to add desired properties, such as, for example, thermoresponsiveness, degradability, hydrophobicity, and adhesiveness. Active agents can also be attached to the polymer backbone using the free hydroxyl groups, or can be attached to pendant groups.

The gelation time of the compositions can be varied from about 0.5 seconds to as long as 10 minutes, and longer if desired. The gelation time will generally be affected by, and can be modified by changing at least the following variables: the initiator system, crosslinker density, macromer molecular weight, macromer concentration (solids content), and type of crosslinker. A higher crosslinker density will provide faster gelation time; a lower molecular weight will provide a slower gelation time. A higher solids content will provide faster gelation time. For redox systems the gelation time can be designed by varying the concentrations of the redox components. Higher reductant and higher oxidant will provide faster gelation, higher buffer concentration and lower pH will provide faster gelation.

The firmness of the formed hydrogel will be determined in part by the hydrophilic/hydrophobic balance, where a higher hydrophobic percent provides a firmer hydrogel. The firmness will also be determined by the crosslinker density (higher density provides a firmer hydrogel), the macromer molecular weight (lower MW provides a firmer hydrogel), and the length of the crosslinker (a shorter crosslinker provides a firmer hydrogel).

The swelling of the hydrogel is inversely proportional to the crosslinker density. Generally, no or minimal swelling is desired, desirably less than about 10 percent.

Elasticity of the formed hydrogel can be increased by increasing the size of the backbone between crosslinks and decreasing the crosslinker density. Incomplete crosslinking will also provide a more elastic hydrogel. Preferably the elasticity of the hydrogel substantially matches the elasticity of the tissue to which the composition is to be administered.

The present invention also provides for a method of marking a biopsy site within a subject's body, comprising depositing an implantable biopsy cavity marking device comprising at least one body comprising a resilient biocompatible material, wherein the marker is radiopaque and echogenic. Generally, the marking device comprises a non-bioabsorbable material. Preferably, the at least one marker comprises a metal marker material, *e.g.,* platinum, iridium, nickel, tungsten, tantalum, gold, silver, rhodium, titanium, alloys thereof, and stainless steel.

Using the method, the biocompatible material comprises a polymer. Preferably, the polymer is one or more polymers selected form the group consisting of polyacrylates, ethylene-vinyl acetate polymers, non-erodible polyurethanes, polystyrenes, polyvinyl chloride, polyvinyl fluoride, poly(vinyl imidazole), chlorosulphonated polyolifins, polyethylene oxide, polyvinyl alcohol, teflon, calcium carbonate, carrageenan and nylon, and derivatives thereof. In another embodiment, the polymer is a polyvinyl alcohol gel, foam or sponge, or alkylation, and acylation derivatives thereof, including esters. In yet another embodiment, the polymer is a hydrogel selected from the group consisting of a crosslinked polyethylene oxide, polypropylene oxide, polyvinyl alcohol, polyvinyl acetate, polyvinyl pyrrolidone, polyhydroxyalkyl acrylate, polystyrene sulfonate and copolymers or combinations thereof.

Generally, the quantity of polymer comprises between about 0.1 ml and about 5 ml of ultrasound-detectable nondegradable material. Preferably, the quantity of polymer comprises between about 0.2 ml and about 2.5 ml of ultrasound-detectable nondegradable material. More preferably, the quantity of polymer comprises between about 0.5 ml and about 1.5 ml of ultrasound-detectable nondegradable material.

In one embodiment, the material is effective to form a gel upon introduction within the body of an animal. Preferably, the material forms a gel upon introduction within the body of an animal after contact with a biocompatible liquid. Generally, the biocompatible liquid comprises a hemostatic agent selected from the group consisting of adrenochrome, algin, alginic acid, aminocaproic acid, batroxobin, carbazochrome salicylate, cephalins, cotarmine, ellagic acid, epinephrine, ethamsylate, factor VIII, factor IX, factor XIII, fibrin, fibrinogen, naphthoquinone, oxamarin, oxidized cellulose, styptic collodion, sulamrin, thrombin, thromboplastin (factor III), tolonium chloride, tranexamic acid, and vasopression.

In another embodiment, the biocompatible liquid comprises a pharmaceutical agent selected from the group consisting of penicillins, cephalosporins, vancomycins, aminoglycosides, quinolones, polymyxins, erythromycins, tetracyclines, streptomycins, sulfa drugs, chloramphenicols, clindamycins, lincomycins, sulfonamides, paclitaxel, docetaxel, acetyl sulfisoxazole, alkylating agents, antimetabolites, plant alkaloids, mechlorethamine, chlorambucil, cyclophosphamide, melphalan, ifosfamide, methotrexate, 6-mercaptopurine, 5-fluorouracil, cytarabine, vinblastine, vincristine, etoposide, doxorubicin, daunomycin, bleomycin, mitomycin, carmustine, lomustine, cisplatin, interferon, asparaginase, tamoxifen, flutamide, amantadines, rimantadines, ribavirins, idoxuridines, vidarabines, trifluridines, acyclovirs, ganciclovirs, zidovudines, foscarnets, interferons, prochlorperzine edisylate, ferrous sulfate, aminocaproic acid, mecamylamine hydrochloride, procainamide hydrochloride, isoproterenol sulfate, phenmetrazine hydrochloride, bethanechol chloride, methacholine chloride, isopropamide iodide, tridihexethyl chloride, phenformin hydrochloride, methylphenidate hydrochloride, theophylline cholinate, cephalexin hydrochloride, diphenidol, meclizine hydrochloride, prochlorperazine maleate, phenoxybenzamine, thiethylperzine maleate, anisindone, diphenadione erythrityl tetranitrate, isoflurophate, acetazolamide, methazolamide, bendroflumethiazide, chloropromaide, tolazamide, chlormadinone acetate, phenaglycodol, allopurinol, aluminum aspirin, hydrocortisone, hydrocorticosterone acetate, cortisone acetate, dexamethasone and its derivatives such as betamethasone, triamcinolone, methyltestosterone, 17-S-estradiol, ethinyl estradiol, ethinyl estradiol 3-methyl ether, prednisolone, 17-hydroxyprogesterone acetate compounds, 19-nor-progesterone, norgestrel, norethindrone, norethisterone, norethiederone, progesterone, norgesterone, norethynodrel, aspirin, indomethacin, naproxen, fenoprofen, sulindac, indoprofen, nitroglycerin, isosorbide dinitrate, propranolol, timolol, atenolol, alprenolol, cimetidine, clonidine, imipramine, dihydroxyphenylalanine, theophylline, calcium gluconate, ketoprofen, ibuprofen, cephalexin, haloperidol, zomepirac, ferrous lactate, vincamine, diazepam, phenoxybenzamine, milrinone, capropril, mandol, quanbenz, hydrochlorothiazide, ranitidine, flurbiprofen, fenufen, fluprofen, tolmetin, alclofenac, mefenamic, flufenamic, difuinal, nizatidine, sucralfate, etintidine, tetratolol, minoxidil, chlordiazepoxide, diazepam, amitriptyline, imipramine, prostaglandins, coagulation factors, analogs of these compounds, derivatives of these compounds, and pharmaceutically acceptable salts of these compounds, analogs and derivatives.

In another embodiment, the biocompatible liquid comprises a hemostatic agent selected from the group consisting of adrenochrome, algin, alginic acid, aminocaproic acid, batroxobin, carbazochrome salicylate, cephalins, cotarmine, ellagic acid, epinephrine, ethamsylate, factor VIII, factor IX, factor XIII, fibrin, fibrinogen, naphthoquinone, oxamarin, oxidized cellulose, styptic collodion, sulamrin, thrombin, thromboplastin (factor III), tolonium chloride, tranexamic acid, and vasopression.

In another embodiment, the biocompatible liquid comprises a pharmaceutical agent selected from the group consisting of penicillins, cephalosporins, vancomycins, aminoglycosides, quinolones, polymyxins, erythromycins, tetracyclines, streptomycins, sulfa drugs, chloramphenicols, clindamycins, lincomycins, sulfonamides, paclitaxel, docetaxel, acetyl sulfisoxazole, alkylating agents, antimetabolites, plant alkaloids, mechlorethamine, chlorambucil, cyclophosphamide, melphalan, ifosfamide, methotrexate, 6-mercaptopurine, 5-fluorouracil, cytarabine, vinblastine, vincristine, etoposide, doxorubicin, daunomycin, bleomycin, mitomycin, carmustine, lomustine, cisplatin, interferon, asparaginase, tamoxifen, flutamide, amantadines, rimantadines, ribavirins, idoxuridines, vidarabines, trifluridines, acyclovirs, ganciclovirs, zidovudines, foscarnets, interferons, prochlorperzine edisylate, ferrous sulfate, aminocaproic acid, mecamylamine hydrochloride, procainamide hydrochloride, isoproterenol sulfate, phenmetrazine hydrochloride, bethanechol chloride, methacholine chloride, isopropamide iodide, tridihexethyl chloride, phenformin hydrochloride, methylphenidate hydrochloride, theophylline cholinate, cephalexin hydrochloride, diphenidol, meclizine hydrochloride, prochlorperazine maleate, phenoxybenzamine, thiethylperzine maleate, anisindone, diphenadione erythrityl tetranitrate, isoflurophate, acetazolamide, methazolamide, bendroflumethiazide, chloropromaide, tolazamide, chlormadinone acetate, phenaglycodol, allopurinol, aluminum aspirin, hydrocortisone, hydrocorticosterone acetate, cortisone acetate, dexamethasone and its derivatives such as betamethasone, triamcinolone, methyltestosterone, 17-S-estradiol, ethinyl estradiol, ethinyl estradiol 3-methyl ether, prednisolone, 17-hydroxyprogesterone acetate compounds, 19-nor-progesterone, norgestrel, norethindrone, norethisterone, norethiederone, progesterone, norgesterone, norethynodrel, aspirin, indomethacin, naproxen, fenoprofen, sulindac, indoprofen, nitroglycerin, isosorbide dinitrate, propranolol, timolol, atenolol, alprenolol, cimetidine, clonidine, imipramine, dihydroxyphenylalanine, theophylline, calcium gluconate, ketoprofen, ibuprofen, cephalexin, haloperidol, zomepirac, ferrous lactate, vincamine, diazepam, phenoxybenzamine, milrinone, capropril, mandol, quanbenz, hydrochlorothiazide, ranitidine, flurbiprofen, fenufen, fluprofen, tolmetin, alclofenac, mefenamic, flufenamic, difuinal, nizatidine, sucralfate, etintidine, tetratolol, minoxidil, chlordiazepoxide, diazepam, amitriptyline, imipramine, prostaglandins, coagulation factors, analogs of these compounds, derivatives of these compounds, and pharmaceutically acceptable salts of these compounds, analogs and derivatives.

Generally, the quantity of ultrasound-detectable material comprises a slurry of ultrasound-detectable material in a biocompatible liquid. In another embodiment, the slurry is formed within a delivery tube. In another embodiment, the slurry is formed within a syringe.

In one embodiment, the device is positioned by a positioning step carried out by at least one of: injecting a flowable polymer through a hollow member; pushing a nonflowable polymer through a hollow member; and guiding a solid polymer to the target site. In another embodiment, the flowable polymer injecting step is carried out using a biopsy needle. In another embodiment, the method further comprises the step of changing the polymer from a pre-delivery state prior to the positioning step to a post-delivery state after the positioning step. Generally, the changing step is carried out by at least one of the following: hydration, changing temperature, electrical stimulation, magnetic stimulation, chemical reaction with a first additional material, physical interaction with a second additional material, ionization, absorption and adsorption.

In another embodiment, the method further comprises the step of placing a marker element at a generally central location within the polymer at the target site. Generally, the placing step takes place simultaneously with the positioning step. In one embodiment, the placing step is carried out using a radiopaque marker element. In another embodiment, the biopsy site relocating step comprises the step of remotely visualizing the marker element.

In another embodiment, the method further comprises: testing the tissue sample and, if the testing indicates a need to do so, medically treating the biopsy site. Generally, the medically treating step comprises activating an agent carried by the polymer. Preferably, the activating step is carried out by at least one of: injecting a radiation-emitting element at the vicinity of the target site; externally irradiating the target site; and providing a triggering substance to the agent. In another embodiment, the medically treating step comprises delivering a therapeutic agent to the target site. In another embodiment, the delivering step is carried out using at least one of: a chemotherapy agent; a radiation-emitting element; thermal energy; ionization energy; gene therapy; vector therapy; electrical therapy; vibrational therapy; and anti-angiogenesis.

In another embodiment, the method further comprises the step of relocating the biopsy by finding the polymer. Preferably, the relocating step is carried out prior to the medically treating step. More preferably, the medical treating step comprises removal of tissue.

Although this invention has been described in connection with its most preferred embodiment, additional embodiments are within the scope and spirit of the claimed invention. The preferred marker of this invention is intended merely to illustrate the invention, and not limit the scope of the invention as it is defined in the claims that follow.

In addition, information regarding procedural or other details supplementary to those set forth herein is described in cited references specifically incorporated herein by reference.

It would be obvious to those skilled in the art that modifications or variations may be made to the preferred embodiment described herein without departing from the novel teachings of the present invention. All such modifications and variations are intended to be incorporated herein and within the scope of the claims.

## Claims

1. An implantable biopsy cavity marking device comprising at least one body comprising a resilient biocompatible material, wherein the marking device is radiopaque and echogenic.

2. The device of claim 1 wherein the at least one body comprises a non-bioabsorbable material.

3. The device of claim 1 wherein the marking device further comprises an X-ray detectable object of specific predetermined non-biological configuration embedded in the body of the marking device.

4. The device of claim 2 wherein the X-ray detectable object comprises a material selected from the group consisting of platinum, iridium, nickel, tungsten, tantalum, gold, silver, rhodium, titanium, alloys thereof, and stainless steel.

5. The device of claim 4 wherein the biocompatible material comprises a polymer.

6. The device of claim 5 wherein the polymer is one or more polymers selected form the group consisting of polyacrylates, ethylene-vinyl acetate polymers, non-erodible polyurethanes, polystyrenes, polyvinyl chloride, polyvinyl fluoride, poly(vinyl imidazole), chlorosulphonated polyolifins, polyethylene oxide, polyvinyl alcohol, teflon, calcium carbonate, carrageenan and nylon, and derivatives thereof.

7. The device of claim 5 wherein the polymer is selected form the group consisting of a polyvinyl alcohol gel, foam or sponge, hydrogel, and esters and acylation derivatives thereof.

8. The device of claim 7 wherein the polymer is a hydrogel selected from the group consisting of a crosslinked polyethylene oxide, polypropylene oxide, polyvinyl alcohol, polyvinyl acetate, polyvinyl pyrrolidone, polyhydroxyalkyl acrylate, polystyrene sulfonate and copolymers or combinations thereof..

9. The device of claim 4 wherein the biocompatible material comprises a polymer having a radiopaque additive.

10. The device of claim 5 wherein the radiopaque additive is selected from the group consisting of barium-containing compounds, bismuth-containing compounds, powdered tantalum, powdered tungsten, barium carbonate, bismuth oxide, and barium sulfate.
